# EUROPÄISCHE PATENTSCHRIFT

(11) **EP 0 911 311 B1**
(45) Veröffentlichungstag und Bekanntmachung des Hinweises auf die Patenterteilung: **07.05.2003**
(21) Anmeldenummer: 98119473.1
(22) Anmeldetag: 15.10.1998
(51) Int. Cl.: C07C 33/12, C07C 29/00, C07C 47/02, C07C 49/203, C07C 49/24, C07F 9/54, C07F 7/08

(54) **Herstellung eines Lycopinmetaboliten**
Preparation of lycopene metabolites
Préparation de métabolites de lycopène

(30) Priorität: 20.10.1997 EP 97118144; 13.08.1998 EP 98115249
(43) Veröffentlichungstag der Anmeldung: 28.04.1999
(73) Patentinhaber: F. HOFFMANN-LA ROCHE AG, 4070 Basel (CH)
(72) Erfinder: Pfander, Hanspeter, 3006 Bern (CH); Traber, Bruno, 3110 Münsingen (CH)

(56) Entgegenhaltungen:
- EP-A- 0 382 067
- DE-A- 2 554 924

## Beschreibung

Die vorliegende Erfindung betrifft ein mehrstufiges Verfahren zur Herstellung eines oxidativen Metaboliten des Carotinoides Lycopin sowie im Herstellungsverfahren produzierte neue Zwischenprodukte.

Bekanntlich spielen Carotinoide, u.a. Lycopin, eine wichtige Rolle bei der Chemoprevention (Prophylaxe) von Krebs [siehe beispielsweise J.S. Bertram, Pure & Appl. Chem. 66, 1025-1032 (1994) und die darin erwähnten Literaturstellen; N.I. Krinsky, Nat. Antioxid. Health Dis. 1994, 239-261; J.S. Bertram, Oxid. Stress Aging 1995, 221-235; sowie T. Narisawa et al., Cancer Lett. 107(1), 137-142 (1996)], und deren Anwendung bei der klinischen Forschung ist gut etabliert [A. Bendich, Pure & Appl. Chem. 66, 1017-1024 (1994) und die darin erwähnten Literaturstellen]. Levy et al. wiesen die verhütende Wirkung von Lycopin, der Formel 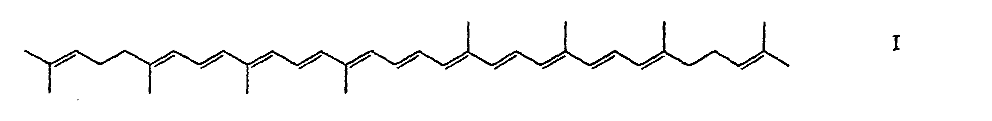 , gegen das Wachstum von menschlichen endometrialischen, Brust- und Lungenkrebszellen nach [Nutr. Cancer, 24, 257-266 (1995)]. In J. Natl. Cancer Inst. 87, 1767-1776 (1995) offenbarten E. Giovannucci et al., dass eine an Lycopin reiche Diät das Risiko von prostatem Krebs reduziert.

Das rote Carotinoid Lycopin kommt u.a. in Tomaten vor. Die Feststellung, dass bezüglich der Wirkung gegen Krebs gekochte Tomaten wesentlich wirksamer sind als rohe, könnte darauf zurückgeführt werden, dass nach dem Kochen das Lycopin eine verbesserte Bioverfügbarkeit aufweist; andererseits könnte es sich bei der biologisch wirksamen Verbindung um ein Oxidationsprodukt oder einen Metaboliten von Lycopin handeln. In neueren Untersuchungen des Carotinoidgehalts menschlichen Blutplasmas wurden neue Lycopinmetaboliten identifiziert, und zwar das 2,6-Cyclolycopin-1,5-diol und vermeintlich das 5,6-Dihydroxy-5,6-dihydrolycopin [F. Khachik et al., J. Cell Biochem. 1995 (Suppl. 22), 236-246 und 11th International Symposium on Carotenoids, Leiden 1996, O.P.1.3; sowie F. Khachik, Book of Abstracts, 213th ACS Nat. Meeting, San Francisco, April 13-14, 1997]. Der ersterwähnte bekannte Metabolit, der Formel 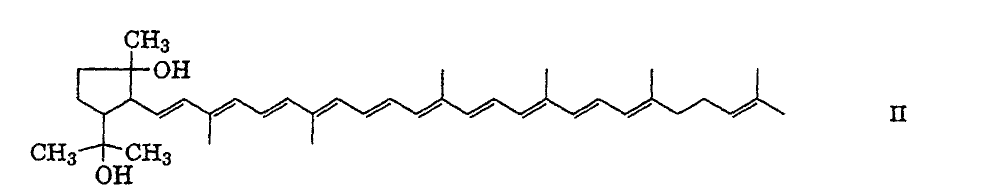 , zeigt Aktivität bei der Krebswachstumsverhütung in menschlichen und Maus-Zellen.

Bisher ist über zwei Synthesen von oxidativen Metaboliten von Lycopin berichtet worden, und zwar in Biosci. Biotechn. Biochem. 59, 2153-2155 (1995; Y. Lu et al.) und am oben erwähnten 11th Int. Symp. on Carotenoids, Leiden 1996 (O.P.3.5; F. Khachik et al.). Dabei handelt es sich um partielle Synthesen, welche jeweils von Lycopin selber ausgehen. Es wurde nun gefunden, dass sich das (bekannte) 2,6-Cyclolycopin-1,5-diol (II) durch ein mehrstufiges Verfahren herstellen lässt, und zwar ausgehend von dem leicht erhältlichen α-Terpinylacetat. Bei diesem Verfahren handelt es sich um die erste Totalsynthese eines oxidativ erzeugten Metaboliten von Lycopin.

Die Erfindung betrifft daher ein Verfahren zur Herstellung von 2,6-Cyclolycopin-1,5-diol (II), welches dadurch gekennzeichnet ist, dass man α-Terpinylacetat der Formel 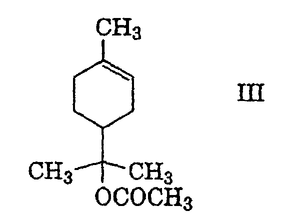 zu 4-(1-Acetoxy-1-methylethyl)-1-methyl-cyclohexan-1,2-diol der Formel 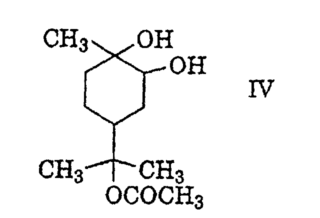 [Cyclohexandiol (IV)] oxidativ dihydroxyliert, das Cyclohexandiol (IV) zu 3-(1-Acetoxy-1-methylethyl)-6-oxo-heptanal der Formel 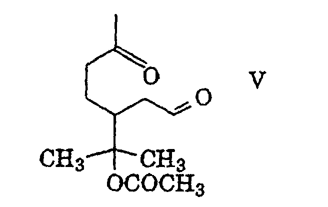 [Ketoaldehyd (V)] oxidativ spaltet, den Ketoaldehyd (V) einer intramolekularen Aldolkondensation zu 3-(1-Acetoxy-1-methylethyl)-2-formyl-1-methylcyclopentanol der Formel 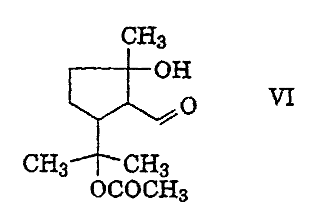 [Cyclopentanol (VI)] unterwirft, das Cyclopentanol (VI) zu 3-(1-Acetoxy-1-methylethyl)-2-formyl-1-methyl-1-trimethylsilyloxy-cyclopentan der Formel 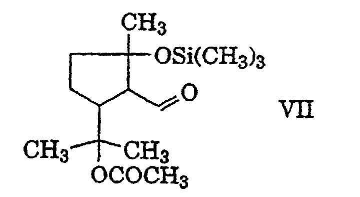 [Formylcyclopentan (VII)] silyliert, das Formylcyclopentan (VII) einer C₃-Kettenverlängerung mit Aceton sowie gleichzeitig einer Verseifung zur Abspaltung der Acetylgruppe zu 4-[5-(1-Hydroxy-1-methylethyl)-2-methyl-2-trimethylsilyloxy-cyclopentyl]-3-buten-2-on der Formel 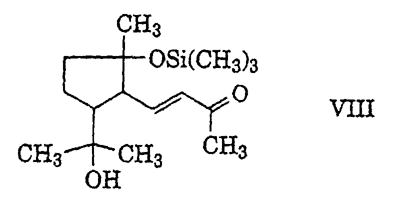 [Cyclopentylbutenon (VIII)] unterwirft, das Cyclopentylbutenon (VIII) mit Vinylmagnesiumbromid zu 5-[5-(1-Hydroxy-1-methylethyl)-2-methyl-2-trimethylsilyloxy-cyclopentyl]-3-methyl-penta-1,4-dien-3-ol der Formel 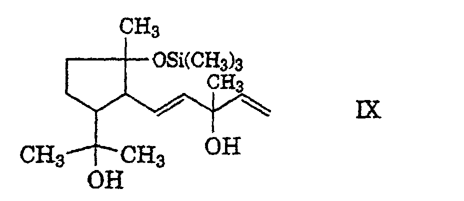 [Pentadienol (IX)] umsetzt, das Pentadienol (IX) unter Entschützung der silylierten Hydroxygruppe in das (5-[2-Hydroxy-5-(1-hydroxy-1-methylethyl)-2-methyl-cyclopentyl]-3-methyl-penta-2,4-dienyl)triphenylphosphoniumsalz der Formel 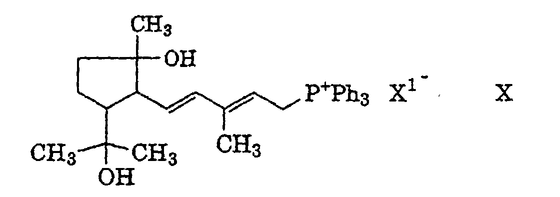 , worin Ph Phenyl und X¹⁻ Halogenid oder Hydrogensulfat bedeuten,
[Phosphoniumsalz (X)] überführt, das Phosphoniumsalz (X) mit 2,7-Dimethyl-2,4,6-octatrien-1,8-dial der Formel 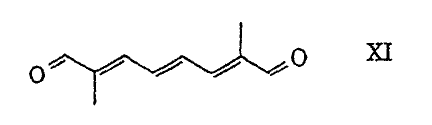 [C₁₀-Dial (XI)] einer Wittig-Reaktion zu 2,7,11-Trimethyl-13-[2-hydroxy-5-(1-hydroxy-1-methylethyl)-2-methyl-cyclopentyl]-trideca-2,4,6,8,10,12-hexaenal der Formel 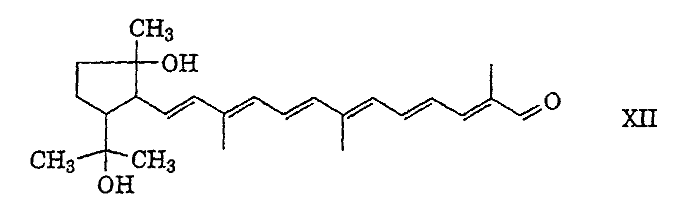 [Tridecahexaenal (XII)] unterwirft und das Tridecahexaenal (XII) mit einem (3,7,11-Trimethyl-dodeca-2,4,6,10-tetraenyl)triphenylphosphoniumsalz der Formel 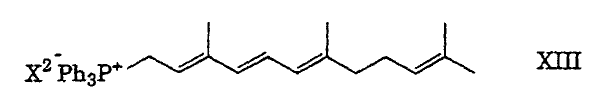 , worin Ph Phenyl und X²⁻ Halogenid oder Hydrogensulfat bedeuten,
[Phosphoniumsalz (XIII)] einer Wittig-Reaktion zum erwünschten 2,6-Cyclolycopin-1,5-diol der Formel II unterwirft.

Ferner betrifft die vorliegende Erfindung die neuen Zwischenprodukte der Formeln V, VI, VII, VIII, IX, X und XII sowie die einzelnen Verfahrensstufen IV → V, V → VI, VI → VII, VII → VIII, VIII → IX, IX → X, X + XI → XII und XII + XIII → II, d.h. die oben definierten einstufigen Verfahren zur Herstellung der neuen Zwischenprodukte und des bekannten Endproduktes II. Nicht nur die Verbindung der Formel III sondern auch noch die Verbindungen der Formeln IV, XI und XIII sind bekannt: siehe u.a. T. Hirata et al., Chem. Lett. 1982, 671-674 [Cyclohexandiol (IV)] sowie US-Patentschrift 5.166.445 und Helv. Chim. Acta 75, 1848-1865 (1992) [Phosphoniumsalz (XIII)].

Die oxidative Dihydroxylierung des α-Terpinylacetats (III) zum Cyclohexandiol (IV) wird zweckmässigerweise unter Verwendung des Oxidationsmittels Kaliumpermanganat in einem flüssigen Reaktionsmedium bei relativ niedrigen Temperaturen durchgeführt. Als Lösungsmittel für das α-Terpinylacetat (III) kommt insbesondere ein polares organisches Lösungsmittel, wie ein aliphatischer oder cyclischer Ether, z.B. Tetrahydrofuran, oder ein niederes (insbesondere C₁₋₆-) Alkanol, z. B. Ethanol, in Frage. Das Kaliumpermanganat seinerseits wird zweckmässigerweise in Wasser gelöst, und zwar geeigneterweise bei einer Konzentration im Bereich von etwa 2 bis etwa 7% (G/V), und in der wässrigen Lösung zur Lösung des α-Terpinylacetats langsam gegeben. Sicherheitshalber - und da die Reaktion unter diesen Bedingungen im allgemeinen gut abläuft - erfolgt die Zugabe bei relativ niedrigen Temperaturen, d.h. zweckmässigerweise im Temperaturbereich von etwa 0°C bis etwa 40°C; wegen der Gefahr einer Ueberoxidation sollte die Temperatur allerdings im unteren Teil dieses Bereiches gehalten werden. Man verwendet zweckmässigerweise etwa 0,8 bis etwa 1,0 Aequivalente (Aeq.) Kaliumpermanganat bezogen auf die Menge Edukt. Vorteilhaft wird zudem während der Zugabe das Reaktionsgemisch heftig gerührt, und auch nach beendeter Zugabe wird geeigneterweise weitergerührt. Auf diese Weise wird die Reaktion normalerweise innert höchstens etwa zwei Stunden beendet, wobei eine Suspension vorliegt und sich das erwünschte Cyclohexandiol (IV) in Lösung befindet. Zur Isolierung dieses Produktes wird die Suspension filtriert, das Filtrat mit einem geeigneten, mit Wasser nicht mischbaren organischen Lösungsmittel, wie einem niederen halogenierten Kohlenwasserstoff, z.B. Methylenchlorid oder Chloroform, einem aliphatischen Ether, z.B. Diethylether oder tert.Butylmethylether, oder einem niederen aliphatischen Ester, z.B. Ethylacetat, extrahiert und die organische Extraktionsphase, z.B. mit wasserfreiem Natrium- oder Magnesiumsulfat, getrocknet und dann unter vermindertem Druck eingedampft. Gewünschtenfalls kann man den festen Rückstand auf übliche Weise reinigen, z.B. durch Umkristallisation oder Säulenchromatographie.

Anstelle einer wässrigen Lösung des Kaliumpermanganats kann in der oxidativen Dihydroxylierung dieses Oxidationsmittel in anderer Form verwendet werden; hierfür kommen unter anderem in Frage Kaliumpermanganat in alkalischer Lösung, insbesondere mit wässriger Alkalihydroxidlösung, z.B. wässriger Natrium- oder Kaliumhydroxidlösung, und Kaliumpermanganat zusammen mit Magnesiumsulfat in ethanolischwässriger Lösung. In beiden Fällen erfolgt die Umsetzung zweckmässigerweise bei niedrigen Temperaturen, z.B. im Bereich von etwa 0°C bis etwa 5°C, wobei ansonsten die Reaktionsführung in an sich bekannter Weise erfolgen kann (siehe Organikum, Seite 261, und W.T. Weber et al., Tetr. Lett. 1972, 4907 ff). Zudem kommen andere Oxidationsmittel als Kaliumpermanganat in Frage, und zwar insbesondere Osmiumtetroxid/Wasserstoffperoxid. Dabei wird typischerweise eine 6-7%ige Lösung von Wasserstoffperoxid in Methanol, tert. Butanol, Aceton oder Eisessig und eine etwa 0,5%ige Lösung von Osmiumtetroxid in demgleichen Lösungsmittel zum α-Terpinylacetat (III) gegeben und das Reaktionsgemisch mehrere Tage gerührt. Für weitere Details wird beispielsweise auf N.A. Milas et al., J.A.C.S. 58, 1302 ff. (1936) verwiesen.

Bei der Umsetzung des Cyclohexandiols (IV) zum Ketoaldehyd (V) handelt es sich um eine Glykolspaltung, wie diese beispielsweise in Tetr. Lett. 28, 2863 ff. (1987) beschrieben ist. Im jetzigen Fall IV → V erfolgt die Glykolspaltung zweckmässigerweise in einem aprotischen polaren oder apolaren, oder sogar in einem protischen polaren, organischen Lösungsmittel bei niedrigen bis mässigen Temperaturen und unter Verwendung von Blei(IV)acetat [Pb(OCOCH₃)₄] als Oxidationsmittel. Bevorzugte Lösungsmittel zu diesem Zweck sind niedere halogenierte aliphatische Kohlenwasserstoffe, z.B. Methylenchlorid; aromatische Kohlenwasserstoffe, z.B. Benzen oder Toluen; bzw. niedere aliphatische Carbonsäuren, z.B. Essigsäure. Die Umsetzung erfolgt geeigneterweise im Temperaturbereich von etwa -20°C bis etwa 50°C, vorzugsweise bei etwa 0°C. Die Menge Bleiacetat liegt zweckmässigerweise zwischen etwa 1,0 und etwa 1,5 Aequivalenten bezogen auf die Menge Edukt. Dieses Mittel kann gewünschtenfalls unmittelbar, d.h. ohne Verdünnung, zu einer Lösung des Cyclohexandiols (IV) im gewählten Lösungsmittel gegeben werden, oder man kann die beiden Reaktionsteilnehmer jeweils im Lösungsmittel gelöst oder suspendiert zusammenbringen, und zwar vorzugsweise unter Einhaltung einer niedrigen Temperatur, insbesondere einer, die unterhalb von etwa 5°C liegt, und auf jeden Fall unter möglichst weitgehendem Ausschluss von Luftfeuchtigkeit. Um die das Bleiacetat fast unvermeidlich begleitende Essigsäure zu neutralisieren, wird vorteilhaft vor der Bleiacetatzugabe die Lösung oder Suspension des Cyclohexandiols mit wasserfreiem Natriumcarbonat oder mit an einer anderen, eher schwach, anorganischen Base versetzt; zu diesem Zweck wird vorzugsweise vermösertes oder feinkristallines, wasserfreies Natriumcarbonat verwendet. Zudem empfiehlt es sich, das Reaktionsgemisch zu rühren. Auf diese Weise wird die Reaktion normalerweise innert etwa zwei Stunden beendet.

Zur Aufarbeitung des nach der Reaktion erhaltenen Gemisches wird geeigneterweise diesem Gemisch Wasser zugegeben, und dabei lässt man dessen Temperatur auf Raumtemperatur ansteigen. Nach Abfiltration von verbleibenden festen Anteilen und Abtrennung der das Produkt enthaltenden organischen Phase kann gewünschtenfalls in der wässrigen Phase verbleibendes Produkt durch Extrahieren mit weiterem organischem Lösungsmittel, z.B. Methylenchlorid, gewonnen werden. Eine übliche Behandlung der (gesamten) organischen Phase (Trocknen über z.B. wasserfreiem Natrium- oder Magnesiumsulfat, Eindampfen und gewünschtenfalls säulenchromatographische Reinigung) liefert den gewünschten Ketoaldehyd (V).

Die nächste Verfahrensstufe betrifft die intramolekulare Aldolkondensation des Ketoaldehyds (V) zum Cyclopentanol (VI). Diese Kondensation wird zweckmässigerweise durchgeführt, indem man den Ketoaldehyd in einem organischen Lösungsmittel oder sogar in Wasser bei Temperaturen im Bereich von etwa 0°C bis zur Rückflusstemperatur des Reaktionsgemisches, vorzugsweise bei Temperaturen zwischen der Raumtemperatur und etwa 50°C, und in Gegenwart einer Base und auch noch einer organischen Säure reagieren lässt. Als organische Lösungsmittel eignen sich vor allem niedere aliphatische und cyclische Ether, z.B. Diethylether, tert.Butylmethylether bzw. Tetrahydrofuran; niedere aliphatische Ketone, z.B. Aceton; sowie aromatische Kohlenwasserstoffe, z.B. Benzen und Toluen. Es eignen sich als Basen im allgemeinen Amine, wie beispielsweise Dialkylund Trialkylamine, und Stickstoff aufweisende heterocyclische Verbindungen, z.B. Piperidin und Pyrrolidin. In Frage kommende Säuren sind u.a. niedere aliphatische Carbonsäuren, z.B. Essigsäure, und Sulfonsäuren, z.B. p-Toluolsulfonsäure. Sowohl die Base als auch die Carbonsäure kann in katalytischer (bis etwa 0,02-molaren) bis etwa äquimolarer Menge bezogen auf die Menge Edukt verwendet werden. Normalerweise ist die Kondensation innert höchstens 100 Stunden zu Ende, wobei beobachtet wird, dass spätestens nach etwa 24 Stunden ein Gleichgewicht mit einem Produkt:Edukt-Verhältnis von etwa 1:1 erreicht wird.

Auch im Falle dieser intramolekularen Aldolkondensation kann das so produzierte Cyclopentanol (VI) in an sich bekannter Weise vom Reaktionsgemisch isoliert und gewünschtenfalls gereinigt werden, insbesondere durch Waschen mit wässriger basischer und/oder mineralsaurer Lösung, z.B. wässriger Natriumcarbonat-, Salzsäure- und/oder Natriumchloridlösung, Extrahieren mit einem geeigneten organischen Lösungsmittel, beispielsweise mit einem Ether, z.B. tert.Butylmethylether, Abtrennen und Trocknen der organischen Phase, Eindampfen dieser Phase und gewünschtenfalls noch Umkristallisation und/oder säulenchromatographische Reinigung des festen Rückstandes.

Silylierungen zum Schützen einer Hydroxylgruppe sind u.a. auf dem Gebiet der Carotinoide - wie bei der Silylierung des Cyclopentanols (VI) zum Formylcyclopentan (VII) im vorliegenden mehrstufigen Herstellungsverfahren - besonders geläufige Reaktionsstufen, über welche zahlreiche Publikationen existieren [siehe beispielsweise F. Kienzle und R. E. Minder, Helv. Chim. Acta 61, 242 (1978), A. Haag und C. H. Eugster , ibid. 65, 1795 (1982), sowie D. J. Buschor und C. H. Eugster, ibid. 73, 1002 (1990)]. Nicht nur die Trimethylsilylgruppe, sondern auch andere Schutzgruppen sind denkbar, vorausgesetzt, dass sie stabil gegenüber Enolaten und Grignard-Reagenzien und gleichzeitig säurestabil sind. Zu ihnen gehören weitere Trialkylsilyl-, Methoxymethyl-, Methoxyethoxymethyl- und Tetrahydropyranylschutzgruppen.

Im jetzigen Fall erweist es sich als zweckmässig, die Silylierung (mit einer Trimethylsilylschutzgruppe) unter Verwendung von Trimethylchlorsilan als Silylierungsmittel und einem aprotischen polaren organischen Lösungsmittel durchzuführen. Es wird zudem wie üblich eine Base verwendet. Als Lösungsmittel eignen sich insbesondere niedere, halogenierte aliphatische Kohlenwasserstoffe, z.B. Methylenchlorid; Stickstoff-aufweisende heterocyclische Verbindungen, z.B. Pyridin; niedere aliphatische und cyclische Ether, z.B. Diethylether bzw. Tetrahydrofuran; niedere aliphatische Amine, z.B. Triethylamin; und niedere aliphatische Amide, z.B. Dimethylformamid. Geeignete Basen sind u.a. niedere aliphatische Amine, z.B. Triethylamin; aromatische Amine, z.B. Dimethylanilin; und Stickstoff aufweisende, gegebenenfalls aminierte heterocyclische Verbindungen, z.B. Imidazol und 4-Dimethylamino-pyridin. Wie ersichtlich, können die Amine sowohl als Lösungsmittel wie auch als Basen dienen. Man verwendet gegenüber der Menge Edukt (basierend auf 1 Aequivalent) zweckmässigerweise von etwa 2 bis etwa 4 Aeq. Trimethylchlorsilan und von etwa 2 bis etwa 5 Aeq. Base.

In der Praxis erfolgt die Silylierung derart, dass man zu einer Lösung des Cyclopentanols (VI) und der Base im Lösungsmittel das im gleichen Lösungsmittel gelöste Silylierungsmittel gibt, und zwar bei Temperaturen im Bereich von etwa -10°C bis zur Raumtemperatur. Zudem erfolgt die Zugabe zweckmässigerweise unter einem inerten Schutzgas, z.B. Stickstoff, um möglichst Luftfeuchtigkeit auszuschliessen, sowie unter Rühren. Die Silylierung ist unter den obigen Bedingungen normalerweise innert etwa 24 Stunden beendet. Die Aufarbeitung des nach der Reaktion erhaltenen Gemisches kann auf gängige Weise erfolgen, z.B. durch Abfiltration der festen Anteile, Eindampfen des Filtrats und Reinigung des festen Rückstandes, beispielsweise durch Umkristallisation und/oder Säulenchromatographie, um zum mehr oder weniger reinen Formylcyclopentan (VII) zu gelangen.

Die nächste Verfahrensstufe, d.h. die C₃-Kettenverlängerung mit Aceton und gleichzeitig Verseifung des Formylcyclopentans (VII) zum Cyclopentylbutenon (VIII), wird zweckmässigerweise durchgeführt, indem man zunächst ein Lithiumdialkylamid (als Base) aus einem Lithium alkyl, z.B. n-Butyllithium, und einem sekundären Amin, insbesondere einem Di(C₁₋₆alkyl)amin, z.B. Diisopropylamin, frisch herstellt und mit Aceton in einem geeigneten organischen Lösungsmittel, insbesondere einem aprotischen polaren, zum Enolat des Acetons umsetzt; dann wird das Enolat mit dem Formylcyclopentan (VII) umgesetzt. Als organisches Lösungsmittel für die "in situ"-Lithiumdialkylamidherstellung eignet sich im allgemeinen ein aprotisches, wie ein niederer aliphatischer oder cyclischer Ether, z.B. Diethylether bzw. Tetrahydrofuran, oder ein aromatischer Kohlenwasserstoff, z.B. Toluen. Diese Herstellung erfolgt zudem zweckmässigerweise bei relativ niedrigen Temperaturen, insbesondere im Bereich von etwa -10°C bis etwa +10°C, vorzugsweise bei etwa 0°C, unter einem inerten Schutzgas, z.B. Stickstoff, und unter Rühren. Das Lithiumalkyl und das sekundäre Amin werden geeigneterweise in etwa äquimolaren Mengen eingesetzt. Nach einer ausreichenden Reaktionsdauer, die normalerweise bis etwa eine Stunde beträgt, wird zweckmässigerweise bis auf etwa -70°C abgekühlt und anschliessend das Aceton in dem gleichen Lösungsmittel zugegeben. Dabei wird zweckmässigerweise ein deutlicher Ueberschuss an der Base Lithiumdialkylamid, insbesondere von etwa 1,1 bis etwa 2 Aequivalenten, gegenüber dem Aceton (1 Aeq.) verwendet, um die Selbstkondensation des Acetons möglichst zu unterdrücken. Nach einer kurzen Rührperiode bei der tiefen Temperatur wird das Formylcyclopentan (VII) zugegeben, und zwar zweckmässigerweise in einer etwas niedrigeren molaren Menge als die Menge Aceton. Unter Erwärmen des Reaktionsgemisches auf etwa -20°C bis etwa 0°C reagiert das Aceton mit dem Formylcyclopentan (VII), und zwar im erwähnten Temperaturbereich relativ rasch. Zur Isolierung und Reinigung des so hergestellten Cyclopentylbutenons (VIII) kann man beispielsweise dem Gemisch gesättigte wässrige Ammoniumchloridlösung zugeben, die organische Phase abtrennen und mit Wasser und/oder gesättigter wässriger Natriumchloridlösung waschen, über einem Trockenmittel, wie beispielsweise wasserfreiem Natrium- oder Magnesiumsulfat, trocknen, und die organische Phase schliesslich eindampfen; eine (weitere) Reinigung des erhaltenen Rückstandes kann gewünschtenfalls vorgenommen werden, beispielsweise durch Umkristallisation und/oder Säulenchromatographie.

Bei der anschliessenden Verfahrensstufe handelt es sich um eine Grignard-Reaktion. Das Cyclopentylbutenon (VIII) und das Vinylmagnesiumbromid werden zweckmässigerweise in einem aprotischen, polaren, organischen Lösungsmittel, wie einem niederen aliphatischen oder cyclischen Ether, z.B. Diethylether oder Dimethoxyethan bzw. Tetrahydrofuran, Tetrahydropyran oder Dioxan, oder einem Amid, z.B. Hexamethylphosphortriamid, und im Temperaturbereich von etwa -50°C bis etwa 0°C, vorzugsweise von etwa -40°C bis etwa -20°C, miteinander reagiert. Pro Aequivalent Cyclopentylbutenon (VIII) verwendet man zweckmässigerweise etwa 2 bis 4 Aequivalente Vinylmagnesiumbromid. Der Zusatz eines aliphatischen Amins, z.B. Triethylamin, dient zur Erhöhung der Reaktivität. Die Isolierung und Reinigung des so erhaltenen Pentadienols (IX) kann analog der im Zusammenhang mit der vorhergehenden Verfahrensstufe VII → VIII beschriebenen Aufarbeitung durchgeführt werden.

Als zusätzliche Massnahme im erfindungsgemässen Herstellungsverfahren kann unmittelbar vor der Verfahrensstufe VIII → IX die freie tertiäre Hydroxylgruppe des Cyclopentylbutenons (VIII) geschützt werden, und zwar zweckmässigerweise durch Trimethylsilylierung analog der Verfahrensstufe VI → VII; nach der entsprechend modifizierten Verfahrensstufe VIII → IX kann die silylierte Hydroxylgruppe auf konventionelle Weise entschützt werden, was wiederum das Pentadienol (IX) liefert.

Die anschliessende Phosphoniumsalzbildung IX → X wird zweckmässigerweise durchgeführt, indem man eine Lösung des Pentadienols (IX) und eines Triphenylphosphoniumhalogenids bzw. von Triphenylphosphoniumhydrogensulfat in einem polaren organischen Lösungsmittel mehrere Stunden rührt. Als solche Lösungsmittel eignen sich insbesondere niedere aliphatische Alkohole, z.B. Methanol und Ethanol; und niedere, halogenierte aliphatische Kohlenwasserstoffe, z.B. Methylenchlorid und Chloroform. Pro Aequivalent Pentadienol (IX) verwendet man geeigneterweise zwischen etwa 1 und etwa 1,2 Aequivalenten Triphenylphosphoniumhalogenid bzw. -hydrogensulfat. Bei diesem Triphenylphosphoniumsalz handelt es sich vorzugsweise um ein Halogenid, insbesondere um das Chlorid oder Bromid, wobei Triphenylphosphoniumbromid ganz bevorzugt eingesetzt wird. Die Umsetzung wird zweckmässigerweise im Temperaturbereich von etwa 0°C bis etwa 50°C, vorzugsweise bei der Raumtemperatur, durchgeführt und dauert in der Regel von etwa 12 bis etwa 72 Stunden. Die Isolierung und - falls erwünscht - Reinigung des so erhaltenen Phosphoniumsalzes (X) kann nach an sich bekannten Methoden durchgeführt werden.

Bei der vorletzten und der letzten Verfahrensstufe des erfindungsgemässen mehrstufigen Herstellungsverfahrens handelt es sich jeweils um eine insbesondere in der Carotinoid-Chemie bestens bekannte Wittig-Reaktion. In beiden Fällen können ähnliche Reaktionsbedingungen verwendet werden, wobei für die letzte Verfahrensstufe im allgemeinen drastischere Bedingungen, u. a. höhere Temperaturen, möglich sind. Die beiden Reaktionsteilnehmer werden jeweils zweckmässigerweise in einem protischen oder aprotischen polaren organischen Lösungsmittel in Gegenwart einer Base miteinander umgesetzt. Als solche Lösungsmittel kommen insbesondere niedere aliphatische Alkohole, z.B. Methanol und Ethanol; niedere halogenierte aliphatische Kohlenwasserstoffe, z. B. Methylenchlorid und Chloroform; alicyclische Ether, z.B. Epoxybutan und weitere Oxyrane, und Tetrahydrofuran; Dimethylformamid; und Dimethylsulfoxid in Frage.

Die Umsetzung erfolgt im ersten Fall (X + XI → XII) zweckmässigerweise bei Temperaturen im Bereich von etwa 0°C bis etwa 60°C, vorzugsweise bei der Raumtemperatur, und im zweiten Fall (XII + XIII → II) zweckmässigerweise bei Temperaturen im Bereich von etwa 0°C bis etwa 60°C, vorzugsweise bei etwa 40°C. Zudem empfiehlt es sich, das jeweilige Phosphoniumsalz (X) bzw. (XIII) in einem geringen Ueberschuss einzusetzen, und zwar geeigneterweise in bis zu etwa 10-prozentigem Ueberschuss. Die Aufarbeitung erfolgt zweckmässigerweise durch Verteilung des nach der Umsetzung erhaltenen Gemisches zwischen Wasser und einem aprotischen, polaren organischen Lösungsmittel, wie einem niederen aliphatischen Ether, Ester oder halogenierten Kohlenwasserstoff, z.B. Diethylether, Ethylacetat bzw. Methylenchlorid oder Chloroform , Abtrennung der organischen Phase, Waschen dieser mit gesättigter Natriumchloridlösung, Extrahieren der wässrigen Phase mit weiterem organischen Lösungsmittel, Trocknen der vereinigten organischen Phasen, beispielsweise mit wasserfreiem Natriumoder Magnesiumsulfat, Eindampfen der getrockneten und vom Trockenmittel befreiten organischen Phase und Reinigung des so erhaltenen Festkörpers, z.B. durch Säulenchromatographie und/oder Umkristallisation.

Die Verbindungen XII und II werden nach Säulenchromatographie normalerweise jeweils als E/Z-Isomerengemisch erhalten, aus dem sich das (all-E)-Isomer durch Umkristallisation, z.B. aus Hexan, isolieren lässt. Im allgeminen kann man gewünschtenfalls im ganzen mehrstufigen Verfahren die Isomerie des jeweils erhaltenen Produktes steuern. So kann man ausgehend von dem (4R)-α-Terpinylacetat der Formel III [(4R)-III] der Reihe nach (1RS,2RS,4R)-IV, (3R)-V, (1R,2S,3R)-VI, (1R,2S,3R)-VII, (1'R,2'S,3'R)-VIII, (1'R,2'S,3'R,3RS)-IX, (1'R,2'S,3'R)-X, (1'R,2'S,3'R)-XII und (all-E, 2R,5R,6S)-II herstellen. Die entsprechenden Enantiomeren lassen sich aus (4S)-α-Terpinylacetat herstellen.

Eine Variante des oben definierten und beschriebenen erfindungsgemässen Herstellungsverfahrens besteht darin, dass man das Cyclopentylbutenon (VIII) nicht über das Pentadienol (IX) in das Phosphoniumsalz (X), sondern über zwei alternative Zwischenprodukte ins gleiche Phosphoniumsalz (X) überführt; diese drei Verfahrensstufen beinhaltende Variante besteht insbesondere darin, dass man das Cyclopentylbutenon (VIII) mit einem Phosphonoessigsäure-trialkylester in Gegenwart einer Base einer Horner-Emmons-Olefinierung zum entsprechenden 5-[5-(1-Hydroxy-1-methylethyl)-2-methyl-2-trimethylsilyloxy-cyclopentyl]-3-methyl-penta-2,4-diensäure-alkylester der Formel 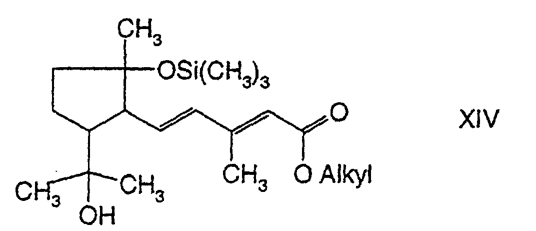 , worin Alkyl C₁₋₆-Alkyl bedeutet,
[Pentadiensäureester (XIV)] unterwirft, den Pentadiensäureester (XIV) unter Entschützung der silylierten Hydroxygruppe zum 5-[2-Hydroxy-5-(1-hydroxy-1-methylethyl)-2-methyl-cyclopentyl]-3-methyl-penta-2,4-dien-1-ol der Formel 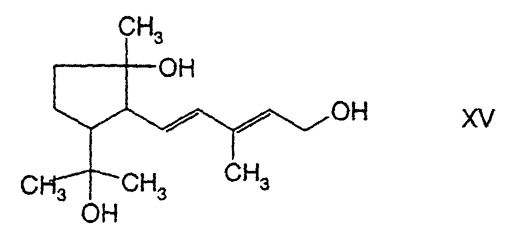 [Pentadienol (XV)] reduziert, und das Pentadienol (XV) in das (5-[2-Hydroxy-5-(1-hydroxy-1-methylethyl)-2-methyl-cyclopentyl]-3-methyl-penta-2,4-dienyl)-triphenylphosphoniumsalz der oben angegebenen Formel X [Phosphoniumsalz (X)] überführt. Der Rest des mehrstufigen Verfahrens zur Herstellung von 2,6-Cyclolycopin-1,5-diol, d.h. die Verfahrensschritte X + XI → XII und XII + XIII → II, erfolgt wie oben definiert und beschrieben. Das so modifizierte, von α-Terpinylacetat ausgehende Verfahren zur Herstellung des Lycopinmetaboliten 2,6-Cyclolycopin-1,5-diol der Formel II stellt einen weiteren Aspekt der vorliegenden Erfindung dar, wie auch noch die in der Variante produzierten neuen Zwischenprodukte der Formeln XIV und XV sowie die einzelnen Verfahrensstufen VIII → XIV, XIV → XV und XV → X, d.h. die oben definierten einstufigen Verfahren zur Herstellung der neuen Zwischenprodukte.

Die Umsetzung des Cyclopentylbutenons (VIII) mit dem Phosphonoessigsäure-trialkylester (Horner-Emmons-Olefinierung) erfolgt zweckmässigerweise in einem niederen aliphatischen Ether oder Diether, z.B. Dimethoxyethan, als Lösungsmittel und in Gegenwart einer starken Base, insbesondere eines Alkalimetallhydrids, z.B. Natriumhydrid; einer Alkalimetallalkoxids, z.B. Natriummethoxid oder -ethoxid; eines Alkyllithiums, z.B. Butyllithium; oder eines Lithiumdialkylamids, z.B. Lithiumdiisopropylamid. Die Umsetzung erfolgt bei niedrigen Temperaturen, und war bei Temperaturen unter etwa -10°C; die untere Grenze liegt bei etwa -60°C. Es erweist sich als praktisch, eine gekühlte Lösung des Phosphonoessigsäuretrialkylesters langsam zu einer ebenfalls gekühlten Suspension der starken Base im gleichen Lösungsmittel unter Rühren und Kühlung zu geben und nach einer Phase Rühren auch noch eine Lösung des Cyclopentylbutenons (VIII) im gleichen Lösungsmittel zugegeben, wobei die Temperatur des jeweiligen Gemisches stets unter etwa -10°C gehalten wird. Zudem empfiehlt es sich, diese Operationen unter einem Inertgas, z.B. Stickstoff oder Argon, durchzuführen. Schliesslich wird das Reaktionsgemisch über mehrere Stunden, beispielsweise 5 bis 15 Stunden, gerührt und allmählich auf Raumtemperatur erwärmen gelassen. Die Isolierung und Reinigung des so erhaltenen Pentadiensäureesters (XIV) kann analog der im Zusammenhang mit der Verfahrensstufe VII → VIII beschriebenen Aufarbeitung durchgeführt werden, wobei allerdings nach der Behandlung mit gesättigter wässriger Ammoniumchloridlösung und vor dem Abtrennen der organischen Phase geeigneterweise ein zusätzliches organisches Lösungsmittel, z.B. Ethylacetat, zum Extrahieren zugegeben wird.

Bei der anschliessenden Verfahrensstufe der Herstellungsverfahrensvariante handelt es sich um die Reduktion der Estergruppe -COOAlkyl des Pentadiensäureesters (XIV) sowie die Entschützung der ebenfalls vorhandenen Trimethylsilyloxygruppe. Die Reduktion wird zweckmässigerweise unter Verwendung eines zu diesem Zweck konventionell eingesetzten Reduktionsmittels, insbesondere eines Metallhydrids, z.B. Diisobutylaluminiumhydrid oder Lithiumaluminiumhydrid, oder eines Alkoxymetallhydrids, durchgeführt. Zudem erfolgt die Umsetzung zweckmässigerweise in einem aliphatischen Kohlenwasserstoff, z.B. Hexan; einem aliphatischen oder cyclischen Ether, z.B. Diethylether bzw. Tetrahydrofuran, einem niederen aliphatischen Alkohol, z.B. Ethanol, oder einem anderen wasserlöslichen organischen Lösungsmittel bei im allgemeinen niedrigen Temperaturen. Im Falle der Verwendung von Diisobutylaluminiumhydrid als Reduktionsmittel erfolgt die Umsetzung beispielsweise bei Temperaturen, die möglichst etwa -40°C nicht übersteigen und in der Regel bei etwa -60°C liegen. Nach dem Versetzen des Pentadiensäureesters (XIV) mit dem Reduktionsmittel kann das Reaktionsgemisch allerdings auf Raumtemperatur erwärmen gelassen und anschliessend die Aufarbeitung ebenfalls analog der im Zusammenhang mit der Verfahrensstufe VII → VIII beschriebenen Aufarbeitung mit eingeschalteter Extraktionsstufe unter Verwendung von beispielsweise Ethylacetat als Extraktionsmittel durchgeführt werden, wobei im Vergleich zu der Aufarbeitung nach der vorhergehenden Verfahrensstufe VIII → XIV die eingedampfte organische Phase auch noch mit einer organischen oder anorganischen Säure als wässrige Lösung, z.B. Salzsäure, versetzt wird (was die Entschützung zustande bringt). Diese Säurebehandlung wird dann zweckmässigerweise von einer Verteilung des Gemisches zwischen Wasser und dem Extraktionsmittel, Trocknen und Eindampfen der (vereinigten) organischen Phase(n) und gewünschtenfalls auch noch Reinigung, z.B. durch Umkristallisation und/oder Säulenchromatographie, gefolgt.

Die darauffolgende Phosphoniumsalzbildung XV → X kann analog der oben beschriebenen Phosphoniumsalzbildung IX → X durchgeführt werden, d.h. für diese Reaktion gelten die gleichen Reaktionsbedingungen.

Wie oben erwähnt, betrifft die vorliegende Erfindung auch die im Herstellungsverfahren (in beiden Varianten) produzierten neuen Zwischenprodukte, d. h.
3-(1-Acetoxy-1-methylethyl)-6-oxo-heptanal der Formel V,
3-(1-Acetoxy-1-methylethyl)-2-formyl-1-methyl-cyclopentanol der Formel VI,
3-(1-Acetoxy-1-methylethyl)-2-formyl-1-methyl-1-trimethylsilyloxycyclopentan der Formel VII,
4-[5-(1-Hydroxy-1-methylethyl)-2-methyl-2-trimethylsilyloxy-cyclopentyl]-3-buten-2-on der Formel VIII,
5-[5-(1-Hydroxy-1-methylethyl)-2-methyl-2-trimethylsilyloxy-cyclopentyl]-3-methyl-penta-1,4-dien-3-ol der Formel IX,
das (5-[2-Hydroxy-5-(1-hydroxy-1-methylethyl)-2-methyl-cyclopentyl]-3-methyl-penta-2,4-dienyl)triphenylphosphoniumsalz der Formel 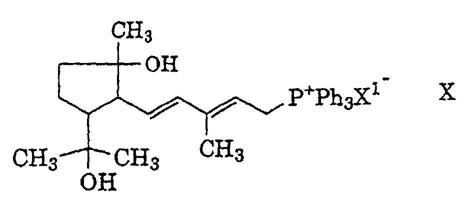 worin Ph Phenyl und X¹⁻ Halogenid oder Hydrogensulfat bedeuten,
2,7,11-Trimethyl-13-[2-hydroxy-5-(1-hydroxy-1-methylethyl)-2-methylcyclopentyl]-trideca-2,4,6,8,10,12-hexaenal der Formel XII,
der 5-[5-(1-Hydroxy-1-methylethyl)-2-methyl-2-trimethylsilyloxy-cyclopentyl]-3-methyl-penta-2,4-diensäure-alkylester der Formel 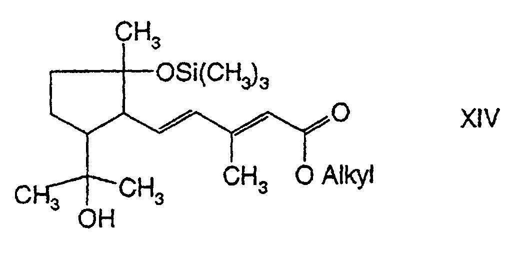 , worin Alkyl C₁₋₆-Alkyl bedeutet,
sowie
5-[2-Hydroxy-5-(1-hydroxy-1-methylethyl)-2-methyl-cyclopentyl]-3-methylpenta-2,4-dien-1-ol der Formel XV,
jeweils als Racemat oder in der diesbezüglichen oben angegebenen optisch aktiven Form, welche ausgehend von (4R)- oder (4S)-α-Terpinylacetat hergestellt werden kann.

Die vorliegende Erfindung wird anhand der nachfolgenden Beispiele veranschaulicht:

### Beispiel 1

### Oxidative Dihydroxylierung III → IV

Eine Lösung von 50 g (255 mmol) α-Terpinylacetat in 800 ml Tetrahydrofuran wurde auf 0°C gekühlt. Unter kräftigem Rühren wurde innert 2 Stunden eine Lösung von 50 g (316 mmol) Kaliumpermanganat in 1 l Wasser zugetropft, und nach Entfernung der Kühlung wurde das Reaktionsgemisch eine weitere Stunde gerührt. Dann wurde das Gemisch durch Celite® (aus Kieselgur verschiedener Korngrössen bestehendes Filterhilfsmittel) filtriert und das Filtrat mit Ethylacetat extrahiert. Man trocknete die organische Phase über wasserfreiem Magnesiumsulfat und dampfte sie anschliessend unter vermindertem Druck ein. Der Rückstand wurde aus der minimalen Menge Ethylacetat und zugegebenem Hexan bei 4°C umkristallisiert und zwecks Erhalten von weiterem Produkt die Mutterlauge säulenchromatographisch mit Silikagel und einem Hexan/Ethylacetat-Gemisch (1:1) gereinigt und der daraus durch Eindampfen erhaltene Rückstand auf gleiche Weise umkristallisiert. Die gesamte Ausbeute an so erhaltenem 4-(1-Acetoxy-1-methylethyl)-1-methyl-cyclohexan-1,2-diol, Smp. 88°C, als weisse Nadeln betrug 33,77 g (148 mmol; 65% der theoretischen Ausbeute; 5,38 g Edukt wurden zurückgewonnen).
¹H-NMR (300 MHz, CDCl₃): 3,36 [dd, J=11,4;4,4, H-C(2)]; 2,3 [br.s., 2x OH]; 2,01 [m, H-C(4)]; 1,94 [s, CH₃COO]; 1,81 [dm, J= 11,4, H-C(5)]; 1,67 [dm, J=11,4, H-C(3)]; 1,40 [m, H₂-C(6)]; 1,39 [s, H₃C(9)]; 1,38 [s, H₃C(10)]; 1,36 [m, H-C(3)]; 1,28 [m, H-C(5)]; 1,23 [H₃C(7)].
¹³C-NMR (75,5 MHz, CDCl₃): 170,57 [C=O]; 84,55 [C(8)]; 75,13 [C(2)]; 70,71 [C(1)]; 44,35 [C(4)]; 37,03 [C(5)]; 31,27 [C(3)]; 27,07 [C(7)]; 23,58 [C(9)]; 23,34 [C(10)]; 22,45 [CH₃COO]; 21,68 [C(6)].
IR (CHCl₃): 3620 w, 3570 w, 3000 m, 2930 m, 1715 s, 1420 w, 1365 s, 1270 s, 1150 m, 1115 m, 1035 m, 1010 m.
MS (EI, 70eV, 250°C): 215 (1, M⁺/-15), 197 (3), 187 (3), 170 (62), 152 (50), 137 (43), 126 (100), 111 (73), 108 (84), 93 (48), 71 (55), 59 (24), 43 (58).

Ausgehend vom optisch aktiven (R)-α-Terpinylacetat erhält man auf obige Weise das Cyclohexandiol (IV) als 1RS,2RS,4R-Diastereomerengemisch, [α]_{D},²³ : -3,3° (c=0,04 in CH₃OH).

### Beispiel 2

### Oxidative Spaltung IV → V

33,77 g (148 mmol) 4-(1-Acetoxy-1-methylethyl)-1-methyl-cyclohexan-1,2-diol und 34,88 g (327 mmol) wasserfreies, fein vermörsertes Natriumcarbonat wurden in 1 l Methylenchlorid vorgelegt, und das Gemisch wurde auf 0°C abgekühlt. Zum Gemisch gab man dann portionenweise 93,3 g eines 85:15-Gemisches von Blei(IV)acetat (156 mmol) und Essigsäure so, dass die Temperatur nicht über 6°C stieg. Das Gemisch wurde eine Stunde gerührt, mit 50 ml Wasser versetzt und auf Raumtemperatur erwärmt. Anschliessend filtrierte man das wässrig-organische Gemisch durch Celite®, trennte vom Filtrat die organische Phase ab, extrahierte die wässrige Phase mit Methylenchlorid, trocknete die vereinigten organischen Phasen über wasserfreiem Magnesiumsulfat, dampfte die getrocknete organische Phase unter vermindertem Druck ein und reinigte den Rückstand säulenchromatographisch unter Verwendung von Silikagel als stationärer Phase und einem 3:2-Gemisch von Hexan und Ethylacetat als Eluierungsmittel.

Auf diese Weise erhielt man 30,14 g (133 mmol) 3-(1-Acetoxy-1-methylethyl)-6-oxo-heptanal als weisses Wachs, Smp. 24°C; die Ausbeute betrug 90% der theoretischen.
¹H-NMR (300 MHz, CDCl₃): 9,72 [dd, J=2,5;1,8, H-C(1)]; 2,58 [ddd, J=16,9;5,8;2,5, H-C(2)]; 2,46 [m, H₂-C(5)]; 2,44 [m, H-C(3)]; 2,26 [ddd, J=16,9;5,8;1,8, H-C(2)]; 2,13 [s, H₃C(7)]; 1,93 [s, CH₃COO]; 1,84 [m, H-C(4)]; 1,53 [s, H₃C(2')]; 1,42 [s, H₃C-C(1')]; 1,37 [m, H-C(4)].
¹³C-NMR (75,5 MHz, CDCl₃): 207,89 [C(6)]; 201,62 [C(1)]; 170,12 [C=O]; 84,40 [C(1')]; 44,96 [C(2)]; 41,99 [C(5)]; 41,88 [C(3)]; 30,04 [C(7)]; 24,17 [C(4)]; 24,15 [C(2')]; 22,39 [CH₃COO]; 22,02 [CH₃-C(1')].
IR (CHCl₃): 3020 m, 2810 w, 2720 w, 1720 s, 1370 s, 1260 s, 1135 m, 1015 m.
MS (EI, 70eV, 150°C): 228 (1,M⁺); 169 (23); 154 (40); 122 (47); 110 (100); 101 (32); 95 (41); 81 (89), 70 (38); 59 (35); 43 (95).

Ausgehend vom 1RS,2RS,4R-Diastereoisomerengemisch des Cyclohexandiols (IV) erhält man auf obige Weise den Ketoaldehyd (V) als 3R-Isomer, [α]_{D},²³ : -6,3° (c=0,28 in CH₃OH).

### Beispiel 3

### Intramolekulare Aldolkondensation V → VI

11,59 g (51,1 mmol) 3-(1-Acetoxy-1-methylethyl)-6-oxo-heptanal wurden zusammen mit 2,3 ml Piperidin, 2,3 ml Essigsäure und 1,15 ml Wasser in 250 ml Tetrahydrofuran gelöst, und die Lösung wurde 21,5 Stunden bei Raumtemperatur gerührt. Man wusch dann die Lösung der Reihe nach mit 5%iger Natriumcarbonatlösung, mit 2N Salzsäure und mit gesättigter Natriumchloridlösung und extrahierte die wässrigen Phasen jeweils mit tert.Butylmethylether. Die vereinigten organischen Phasen wurden mit wasserfreiem Magnesiumsulfat getrocknet und unter vermindertem Druck eingedampft, und der Rückstand wurde dann unter Verwendung von Silikagel als stationärer Phase und einem 13:7-Gemisch von Hexan und Ethylacetat als Eluierungsmittel säulenchromatographisch gereinigt.

Auf diese Weise erhielt man 5,26 g (23,1 mmol) 3-(1-Acetoxy-1-methylethyl)-2-formyl-1-methyl-cyclopentanol als farbloses Oel. Die Ausbeute betrug 45% der theoretischen; da 4,76 g (20,9 mmol, 41%) des eingesetztes Eduktes zurückgewonnen wurden, betrug die Ausbeute an Produkt bezogen auf den Umsatz 77%.
¹H-NMR (300 MHz, CDCl₃): 9,78 [d, J=3,3, HC=O]; 3,04 [td, J=9,9;6,2, H-C(3)]; 2,51 [dd, J=9,9;3,3, H-C(2)]; 2,15-1,96 [m, H-C(4)]; 1,92 [s, CH₃COO]; 1,84 - 1,52 [m, H-C(4), H₂-C(5)]; 1,48 [s, H₃C(2')]; 1,46 [s, H₃C-C(1)]; 1,45 [s, H₃C-C(1')].
¹³C-NMR (75,5 MHz, CDCl₃): 205,5 [HC=O]; 170,2 [O-C=O]; 83,6 [C(1)]; 83,0 [C(1')]; 61,7 [C(2)]; 50,4 [C(3)]; 41,7 [C(4)]; 27,4 [C(2')]; 25,0 [CH₃-C(1')]; 24,9 [C(5)]; 21,8 [CH₃-C(1)], 22,2 [CH₃COO].
IR (CHCl₃): 3610 w, 3000 m, 1720 s, 1460 w, 1375 m, 1270 s, 1215 s, 1130 m, 1020 w.
MS (EI, 70eV, 240°C): 228 (1, M⁺); 168 (19); 153 (32); 123 (37); 110 (92); 95 (42); 81 (87); 69 (29); 59 (30); 43 (100).

Ausgehend vom 3R-Isomeren des Ketoaldehyds (V) erhält man auf obige Weise das Cyclopentanol (VI) als 1R,2S,3R-Isomer, [α]_{D},²⁴ : -4.3° (c=0,38 in CH₃OH).

### Beispiel 4

### Silylierung VI → VII

620 mg (2,72 mmol) 3-(1-Acetoxy-1-methylethyl)-2-formyl-1-methylcyclopentanol und 600 mg (7,5 mmol) Imidazol wurden in 10 ml Methylenchlorid gelöst, und der Lösung wurde eine Lösung von 0,45 ml (3,56 mmol) Trimethylchlorsilan in 5 ml Methylenchlorid bei Raumtemperatur zugespritzt. Man rührte das Reaktionsgemisch 17 Stunden bei dieser Temperatur unter Stickstoff. Zur Aufarbeitung wurde das Gemisch filtriert, das Filtrat unter vermindertem Druck eingedampft und der Rückstand unter Verwendung von Silikagel und einem 17:3-Gemisch von Hexan und Ethylacetat säulenchromatographisch gereinigt.

Auf diese Weise erhielt man 490 mg (1,63 mmol; 60% der theoretischen Ausbeute) 3-(1-Acetoxy-1-methylethyl)-2-formyl-1-methyl-1-trimethylsilyloxycyclopentan als weisses Wachs.
¹H-NMR (300 MHz, CDCl₃): 9,45 [d, J=5, HC=O]; 2,93 [td, J=9,3;6,9 H-C(3)]; 2,19 [dd, J=9,3;4,2, H-C(2)]; 1,95-1,85 [m, H-C(4)]; 1,77 [s, CH₃COO]; 1,59-1,42 [m, H-C(4), H₂-C(5)]; 1,33 [s, H₃C(2')]; 1,32 [s, H₃C-C(1)]; 1,30 [s, H₃C-C(1')]; - 0,02 [s, (CH₃)₃Si].
¹³C-NMR(75,5 MHz, CDCl₃): 205,1 (HC=O); 170,0 (O-C=O); 86,0 [C(1)]; 83,3 [C(1')]; 63,1 [C(2)]; 48,9 [C(3)]; 41,4 [C(4)]; 27,1 [C(2')]; 24,9 [C(5)]; 24,6 [CH₃-C(1')]; 22,0 [CH₃-C(1)]; 21,8 [CH₃COO]; 1,8 [(CH₃)₃Si].
IR (CHCl₃): 2990 m, 1725 s, 1460 w, 1380 m, 1265 s, 1215 s, 1140 m, 1045 m.
MS (EI, 70eV, 150°C): 240 (39); 225 (100); 197 (20); 143 (92); 133 (36); 122 (65); 81 (47); 73 (51); 43 (41).

Ausgehend vom 1R,2S,3R-Isomeren des Cyclopentanols (VI) erhält man auf obige Weise das Formylcyclopentan (VII) als 1R,2S,3R-Isomer, [α]_{D},²⁰ : -12° (c=0,076 in CH₃OH).

### Beispiel 5

### C₃-Kettenverlängerung und Verseifung VII → VIII

275 µl (2 mmol) Diisopropylamin wurden in 8 ml Tetrahydrofuran vorgelegt und 1,25 ml Butyllithium (2 mmol; 1,6M in Hexan) unter Stickstoff bei 0°C zugespritzt. Man rührte das Gemisch 30 Minuten, kühlte es auf -70°C ab und spritzte 110 µl Aceton (1,5 mmol) in 1 ml Tetrahydrofuran zu. Die resultierende Lösung von Lithiumdiisopropylamid wurde 15 Minuten gerührt, und danach spritzte man 300 mg (1 mmol) 3-(1-Acetoxy-1-methylethyl)-2-formyl-1-methyl-1-trimethylsilyloxy-cyclopentan in 1,5 ml Tetrahydrofuran zu. Das Reaktionsgemisch wurde innert 2 Stunden auf 0°C erwärmt und anschliessend mit 10 ml gesättigter Ammoniumchloridlösung sorgfältig versetzt. Die organische Phase wurde abgetrennt, mit Wasser und gesättigter Natriumchloridlösung gewaschen, mit wasserfreiem Magnesiumsulfat getrocknet und schliesslich unter vermindertem Druck eingedampft. Die Reinigung des Rückstandes erfolgte wie üblich durch Säulenchromatographie, und zwar unter Verwendung von Silikagel und einem 3:1-Gemisch von Hexan und Ethylacetat.

Auf diese Weise erhielt man 210 mg (0,7 mmol; 70% der theoretischen Ausbeute) 4-[5-(1-Hydroxy-1-methylethyl)-2-methyl-2-trimethylsilyloxy-cyclopentyl]-3-buten-2-on als farbloses Oel.
¹H-NMR (300 MHz, CDCl₃): 6,83 [dd, J=16,2;9,6, H-C(4)]; 6,01 [d, J=16,2, H-C(3)]; 2,33 [td, J=9,9;5,9, H-C(3')]; 2,21 [s, H₃C(1)]; 2,16 [t, J=9,6, H-C(2')]; 1,98 [m, H-C(4'α)]; 1,83 [m, H-C(5'α)]; 1,61 [m, H-C(4'β), H-C(5'β)]; 1,25 [s, H₃C(2")]; 1,16 [s, H₃C-(1')]; 1,14 [s, H₃C-C(1")]; 0,08 [(H₃C)₃Si].
¹³C-NMR (75,5 MHz, CDCl₃): 198,7 [C(2)]; 152,5 [C(4)]; 132,3 [C(3)]; 85,8 [C(1')]; 72,9 [C(1")]; 56,9 [C(2')]; 54,4 [C(3')]; 40,7 [C(5')]; 28,5 [C(2")]; 27,8 [CH₃-C(1')]; 26,2 [C(1)]; 26,0 [CH₃-C(1")]; 25,4 [C(4')]; 2,2 [(CH₃)₃Si].
IR (CHCl₃): 3440 w, 2980 s, 2375 w, 1730 w, 1675 s, 1620 m, 1385 m, 1255 s, 1050 m, 860 s.
MS (EI, 70eV, 150°C): 298 (2, M⁺); 280 (25); 265 (16); 240 (31); 227 (28); 208 (42); 193 (29); 182 (30); 143 (100); 101 (62); 73 (50); 59 (54); 43 (56).

Ausgehend vom 1R,2S,3R-Isomeren des Formylcyclopentans (VII) erhält man auf obige Weise das Cyclopentylbutenon (VIII) als 1'R,2'S,3'R-Isomer, [α]_{D},²² : -116° (c=0,324 in CH₃OH).

### Beispiel 6

### Grignard-Reaktion VIII → IX

6,6 ml einer 1M Lösung von Vinylmagnesiumbromid (6,6 mmol) in Diethylether wurden in 30 ml Tetrahydrofuran gelöst, und die Lösung wurde dann unter Stickstoff auf -50°C abgekühlt. Man spritzte anschliessend eine Lösung von 490 mg (1,64 mmol) 4-[5-(1-Hydroxy-1-methylethyl)-2-methyl-2-trimethylsilyloxy-cyclopentyl]-3-buten-2-on in 10 ml Tetrahydrofuran langsam zu, rührte das Reaktionsgemisch 30 Minuten, gab weitere 3 ml der etherischen Vinylmagnesiumbromidlösung (3 mmol CH₂=CHMgBr) zu und wärmte das Gemisch auf 0°C.

Zur Aufarbeitung versetzte man das Gemisch mit 20 ml gesättigter Ammoniumchloridlösung, trennte die organische Phase ab, wusch sie mit Natriumchloridlösung, trocknete sie mit wasserfreiem Magnesiumsulfat und dampfte sie unter vermindertem Druck ein. Die Reinigung des Rückstandes erfolgte wie üblich säulenchromatographisch mit Silikagel und einem 17:8-Gemisch von Hexan und Ethylacetat.

Auf diese Weise erhielt man 190 mg (0,58 mmol, 35% der theoretischen Ausbeute) 5-[5-(1-Hydroxy-1-methylethyl)-2-methyl-2-trimethylsilyloxy-cyclopentyl]-3-methyl-penta-1,4-dien-3-ol als farbloses Oel.
¹H-NMR (300 MHz, CDCl₃): 5,91 [dd, J=17,3;10,7, H-C(2)]; 5,65 [dd, J=15,8;8,8, H-C(5)]; 5,53 [d, J=15,8; H-C(4)]; 5,18 [dd, J=17,3;1,1, H-C(1)]; 4,98 [dd, J=10,7;1,1, H-C(1)]; 2,96 [br.s, 2 OH]; 2,19 [m, H-C(3')]; 1,90 [m, H-C(2')]; 1,81 [m, H-C(4'α)]; 1,73 [m, H-C(5'α)]; 1,49 [m, H-C(5'β)]; 1,38 [m, H-C(4'β)]; 1,34 [s, H₃C-C(3)]; 1,17 [s, H₃C-C(1')]; 1,10 [s, H₃C(2"), H₃C-C(1")]; 0,08 [s, (H₃C)₃-Si].
¹³C-NMR (75,5 MHz, CDCl₃): 144,3 [C(2)]; 137,5 [C(4)]; 131,3 [C(5)]; 112,0 [C(3)]; 111,7 [C(1)]; 84,6 [C(1')]; 73,0 [C(1")]; 56,5 [C(2')]; 53,8 [C(3')]; 40,2 [C(5')]; 28,5 [C(2")]; 27,5 [Me-C(3)]; 26,6 [Me-C(1")]; 25,8 [Me-C(1')]; 25,1 [C(4')]; 2,2 [(CH₃)₃Si].
IR (NaCl): 3400 s, 3040 w, 2970 s, 1620 w, 1455 m, 1380s, 1245 s, 1095 s, 1040 s, 835 s.
MS (EI, 70eV, 80°C): 326 (1, M⁺); 308 (28); 293 (13); 241 (40); 223 (89); 218 (72); 197 (37); 173 (81); 143 (100); 117 (28); 73 (53); 57 (23); 43 (44).

Ausgehend vom 1'R,2'S,3'R-Isomeren des Cyclopentylbutenons (VIII) erhält man auf obige Weise das Pentadienol (IX) als 1'R,2'S,3'R,3RS-Isomerengemisch, [α]_{D},²⁵ : -85° (c=0,355 in CH₃OH).

### Beispiel 7

### Entschützung und Phosphoniumsalzbildung IX → X

520 mg (1,6 mmol) 5-[5-(1-Hydroxy-1-methylethyl)-2-methyl-2-trimethylsilyloxy-cyclopentyl]-3-methyl-penta-1,4-dien-3-ol und 600 mg (1,75 mmol) Triphenylphosphoniumbromid wurden in 16 ml eines 1:1-Gemisches von Methanol und Chloroform gelöst, und die Lösung wurde 23 Stunden bei Raumtemperatur unter Stickstoff und unter Lichtausschluss gerührt. Danach wurde eingedampft und der Rückstand, in einer kleinen Menge Methylenchlorid gelöst, in eiskaltem tert.Butylmethylether ausgefällt. Nach Abdekantierung des Ueberstandes und Filtration wurde der gesammelte Niederschlag mit tert.Butylmethylether gewaschen und unter vermindertem Druck getrocknet.

Auf diese Weise erhielt man 1,09 g rohes (5-[2-Hydroxy-5-(1-hydroxy-1-methylethyl)-2-methyl-cyclopentyl]-3-methyl-penta-2,4-dienyl)triphenylphosphoniumbromid. Dieses Produkt wurde ungereinigt im nächsten Verfahrensschritt X + XI → XII (Beispiel 8) eingesetzt.
¹H-NMR und ¹³C-NMR: nicht gemessen
IR (Paraffinöl): 3330 w, 2980 s, 2850 s, 1475 m, 1380 m, 1205 w, 1095 w, 1080 w.
MS (EI, 70eV, 400°C): 463 (2); 277 (12); 262 (100); 183 (63); 153 (9); 108 (22).

Ausgehend vom 1'R,2'S,3'R,3RS-Isomerengemisch des Pentadienols (IX) erhält man auf obige Weise das Phosphoniumsalz (X; Ph = Phenyl, X¹⁻ = Br) als 1'R,2'S,3'R-Isomer, [α]_{D},²⁰ : -18.8° (c=0,085 in CH₃OH).

### Beispiel 8

### Erste Wittigreaktion X + XI → XII

200 mg (maximal 0,36 mmol) rohes (5-[2-Hydroxy-5-(1-hydroxy-1-methylethyl)-2-methyl-cyclopentyl]-3-methyl-penta-2,4-dienyl)triphenylphosphoniumbromid und 50 mg (0,3 mmol) 2,7-Dimethyl-2,4,6-octatrien-1,8-dial wurden in 2 ml Methylenchlorid vorgelegt und mit 1,5 ml 1N Natriumhydroxidlösung versetzt. Das Reaktionsgemisch wurde dann 90 Minuten bei Raumtemperatur gerührt. Zur Aufarbeitung wurde das Gemisch zwischen Methylenchlorid und Wasser verteilt, die wässrige Phase abgetrennt und die organische Phase mit wasserfreiem Magnesiumsulfat getrocknet und unter vermindertem Druck eingedampft. Der Rückstand wurde säulenchromatographisch unter Verwendung von Silikagel und einem 7:3-Gemisch von Hexan und Ethylacetat gereinigt.

Auf diese Weise erhielt man 33 mg (86 µmol, mindestens 28% der theoretischen Ausbeute) 2,7,11-Trimethyl-13-[2-hydroxy-5-(1-hydroxy-1-methylethyl)-2-methyl-cyclopentyl]-trideca-2,4,6,8,10,12-hexaenal in Form eines Gemisches von E/Z-Isomeren als orangefarbenes Pulver. Nach Umkristallisation aus Hexan erhielt man 12 mg (31 µmol; mindestens 10% der theoretischen Ausbeute) dieses Produktes als (all-E)-Isomer.
¹H-NMR (400 MHz, CDCl₃): 9,45 [s, H-C(12')]; 7,02 [dd, J=14,4;11,9, H-C(15)]; 6,95 [d, J=11,9, H-C(14')]; 6,75 [dd, J=15,0; 11,4, H-C(11)]; 6,69 [dd; J=14,4; 11,9, H-C(15')]; 6,37 [d, J=15,0,H-C(12)]; 6,30 [d, J=11,9, H-C(14)]; 6,24 [d, J=15,7;H-C(8)]; 6,16 [d, J=11,4; H-C(10)]; 5,81 [dd, J=15,7;8,9, H-C(7)]; 2,30 [ddd, J=19,7;10.0;6,9, H-C(2)]; 2,24 [dd, J=10,0;8,9, H-C(6)]; 2,03 [s, H₃C(20)]; 1,99 [m, H-C(3α)]; 1,96 [s, H₃C(19)]; 1,88 [s, H₃C(20')]; 1,79 [ddd, J=12,3;8,4;3,8, H-C(4α)]; 1,68 [ddd; J=13,3;10,1;8,4, H-C(4β)]; 1,53 [dtd, J=16,1;6,9;3,8, H-C(3β), 2 OH]; 1,24 [s, H₃C(18)]; 1,18 (s,H₃C(17)]; 1,16 [s,H₃C(16)].
¹³C-NMR (100,6 MHz, CDCl₃): 194,3 [C(12')]; 148,7 [C(14)]; 141,5 [C(13)]; 137,7 [C(8)]; 137,6 [C(15)]; 137,03 [C(13')]; 136,99 [C(12)]; 136,8 [C(9)]; 131,1 [C(14)]; 130,95 [C(10),C(7)]; 127,5 [C(15')]; 127,3 [C(11)]; 82,2 [C(5)]; 73,1 [C(1)]; 55,7 [C(6)]; 54,4 [C(2)]; 40,0 [C(4)]; 28,6 [C(17)]; 27,5 [C(16)]; 26,7 [C(18)]; 25,1 [C(3)]; 13,1 [C(19)]; 13,0 [C(20)]; 9,6 [C(20')].
IR (CHCl₃): 3680 w, 3620 m, 3460 w, 3015 s, 2980 s, 2415 m, 1670 m, 1605 m, 1530 m, 1490 m, 1425 m, 1215 s, 1050 s, 930 m.
MS (EI, 70eV, 270°C): 384 (100, M⁺); 366 (87); 326 (38); 277 (12); 222 (21); 197 (21); 183 (22); 157 (32); 145 (32); 131 (20); 119 (22); 105 (23); 95 (24); 43 (22).
UV/Vis (CH₃COOC₂H₅): 410 nm.

Ausgehend vom 1'R,2'S,3'R-Isomeren des Phosphoniumsalzes (X; Ph = Phenyl, X¹⁻ = Br) erhält man auf obige Weise das Tridecahexaenal (XII) als 1'R,2'S,3'R-Isomer.

### Beispiel 9

### Zweite Wittigreaktion XII + XIII → II

Eine Lösung von 59 mg (0,16 mmol) 2,7,11-Trimethyl-13-[2-hydroxy-5-(1-hydroxy-1-methylethyl)-2-methyl-cyclopentyl]-trideca-2,4,6,8,10,12-hexaenal und 94 mg (0,17 mmol) (3,7,11-Trimethyl-dodeca-2,4,6,10-tetraenyl)-triphenylphosphoniumbromid in 5 ml Methylenchlorid wurde mit 1 ml 1N Natriumhydroxidlösung versetzt und das Reaktionsgemisch 90 Minuten bei der Rückflusstemperatur erhitzt. Zur Aufarbeitung wurde anschliessend die Lösung zwischen Ethylacetat und Wasser verteilt, die wässrige Phase abgetrennt und die organische Phase mit Natriumchloridlösung gewaschen, mit wasserfreiem Natriumsulfat getrocknet und unter vermindertem Druck eingedampft. Der Rückstand wurde säulenchromatographisch unter Verwendung von Silikagel und einem 2:1-Gemisch von Hexan und Ethylacetat gereinigt.

Auf diese Weise erhielt man 38 mg (67 mmol; 43% der theoretischen Ausbeute) 2,6-Cyclolycopin-1,5-diol in Form eines Gemisches von (E/Z)-Isomeren als rotes Pulver. Zur weiteren Reinigung kann dieses beispielsweise aus Hexan umkristallisiert werden, was das (all-E)-Isomer, Smp. 78°C mit Zersetzung, ergibt.
¹H-NMR (300 MHz, CDCl₃): 6,63 [dd, J=15,0;11,1, H-C(11') 6,63 [dd, J=14,9;11,3, H-C(11)]; 6,63 [m, H-C(15), H-C(15')]; 6,51 [dd, J=15,1;11,0, H-C(7')]; 6,36 [d, J=14,9; H-C(12)]; 6,35 [d, J=15,0, H-C(12')]; 6,26 [d, J=15,1, H-C(8')]; 6,25 [d, J=15,7, H-C(8)]; 6,23 [m, H-C(14), H-C(14')]; 6,19 [d, J=11,1, H-C(10')]; 6,16 [d, J=11,3, H-C(10)]; 5,94 [d, J=11,0, H-C(6')]; 5,73 [dd, J=15,7; 9,0, H-C(7)]; 5,16 [m, H-C(2')]; 2,30 [ddd, J=17,1;10,1;7,0, H-C(2)]; 2,23 [dd, J=10,1;9,0, H-C(6)]; 2,12 [m, H₂-C(3'), H₂-C(4')]; 1,98 [s, H₃C(20), H₃C(20')]; 1,97 [s, H₃C(19)]; 1,95 [m, H-C(3α)]; 1,94 [s, H₃C(19')); 1,82 [s, H₃C(18')]; 1,79 [ddd, J=12,3;8,4;3,8, H-C(4α)]; 1,68 [s, H₃C(16')]; 1,67 [m, H-C(4β)]; 1,62 [s, H₃C(17')]; 1,53 [m, H-C(3β)]; 1,24 [s, H₃C(16)]; 1,19 [s, H₃C(18)]; 1,18 [s, H₃C(17)].
¹³C-NMR (75,5 MHz, CDCl₃): 139,5 [C(5')]; 138,2 [C(8)]; 138,0 [C(12)]; 137,3 [C(12')]; 136,7 [C(13')]; 136,3 [C(9')]; 136,2 [C(13)]; 135,4 [C(8')]; 134,9 [C(9)]; 132,9 [C(14)]; 132,5 [C(14')]; 131,8 [C(1')]; 131,6 [C(10')]; 131,5 [C(10)]; 130,3 [C(15)]; 129,9 [C(15')]; 129,4 [C(7)]; 125,7 [C(6')]; 125,2 [C(11')]; 124,8 [C(7')]; 124,6 [C(11)]; 123,9 [C(2')]; 82,2 [C(5)]; 73,1 [C(1)]; 55,6 [C(6)]; 54,3 [C(2)]; 40,2 [C(4')]; 39,7 [C(4)]; 28,5 [C(16)); 27,4 [C(17)]; 26,7 [C(3')+C(18)]; 25,7 [C(16')]; 25,1 [C(3)]; 17,7 [C(17')]; 17,0 [C(18')]; 13,1 [C(19)]; 12,9 [C(19')]; 12,8 [C(20)+C(20')].
IR (CHCl₃): 3640 w, 3600 m, 3440 w, 3010 s, 2980 s, 2860 m, 2390 m, 1515 m, 1470 w, 1415 m, 1220 s, 1045 s.
MS (EI, 70eV, 300°C): 570 (52, M⁺); 552 (2); 478 (14); 464 (12); 223 (19); 209 (25); 159 (52); 145 (62); 133 (36); 105 (62); 91 (43); 69 (31); 55 (20); 43 (100).
UV/Vis (CH₃COOC₂H₅): 491, 459, 433 nm; (Petrolether): 487, 455, 429 nm.

Ausgehend vom 1'R,2'S,3'R-Isomeren des Tridecahexaenals (XII) erhält man auf obige Weise das 2,6-Cyclolycopin-1,5-diol (II) als all-E,2R,5R,6S-Isomer, dessen Circulardichroismus-Daten (CD) wie folgt sind:
CD (Diethylether:Isopentan: Ethanol 5:5:2, -180°C): 216,5 (-3,8, neg. max), 228 (+1,7, pos. max), 244 (+0,2, pos. max), 283,5 (+0,6, pos. max), 297,5 (+3,0, pos. max), 443,5 (-4,9, neg. max), 455 (-3,6, pos. max), 469 (-7,2, neg. max), 498 (-3,4, pos. max), 504 (-8,5, neg. max), 515,5 (-1,4, pos. max).

### Beispiel 10

### Olefinierung VIII → XIV

900 mg einer etwa 55%igen öligen Suspension von Natriumhydrid (etwa 20 mmol) in Petrolether wurden in 30 ml Dimethoxyethan unter Argon vorgelegt, und das Gemisch wurde auf -30°C abgekühlt. Dann wurde eine Lösung von 4,5 ml Phosphonoessigsäure-triethylester (22,5 mmol) in 5,5 ml Dimethoxyethan so zugespritzt, dass die Temperatur stets unter -20°C blieb. Das Gemisch wurde 45 Minuten bei -25°C bis -20°C gerührt und danach eine Lösung von 2 g (6,67 mmol) 4-[5-(1-Hydroxy-1-methylethyl)-2-methyl-2-trimethylsilyloxy-cyclopentyl]-3-buten-2-on (wie in Beispiel 5 beschrieben hergestellt) in 3 ml Dimethoxyethan so zugespritzt, dass die Temperatur unterhalb von -15°C blieb. Man rührte die Lösung etwa 16 Stunden, währenddessen sie sich auf Raumtemperatur erwärmte. Zur Aufarbeitung wurden zur Lösung vorsichtig 10 ml gesättigte Ammoniumchloridlösung zugegeben, und man extrahierte die getrennte wässrige Phase dreimal mit Ethylacetat, wusch die vereinigten organischen Phasen mit gesättigter Natriumchloridlösung, trocknete sie über wasserfreiem Magnesiumsulfat und dampfte sie unter vermindertem Druck ein. Die Reinigung des Rückstandes erfolgte durch Flash-Säulenchromatographie, und zwar unter Verwendung von Silikagel und einem Gemisch von 15-40% Ethylacetat in Hexan.

Auf diese Weise erhielt man 1,19 g [48% der theoretischen Ausbeute; 98%ige Ausbeute bezogen auf umgesetztes Cyclopentylbutenon (VIII), da 1,03 g nicht umgesetztes Ausgangsmaterial blieb] 5-[5-(1-Hydroxy-1-methylethyl)-2-methyl-2-trimethylsilyloxy-cyclopentyl]-3-methyl-penta-2,4-diensäure-ethylester. Das Produkt lag als farbloses Oel vor und bestand aus einem cis/trans-Isomerengemisch des Pentadiensäureesters (XIV).
¹H-NMR (300 MHz, CDCl₃): 7,55 [d,J=6,1,H-C(4)cis]; 6,16 [m,H-C(4)trans, H-C(5)]; 5,71 [s,H-C(2)trans]; 5,62 [s,H-C(2)cis]; 4,13 [m,H₂-C(1''')]; 2,31 [m,H-C(3')]; 2,29 [s,CH₃-C(3)trans]; 2,12 [m,H-C(2')]; 2,00 [s, CH₃-C(3)cis]; 1,96 [m,H-C(4')]; 1,80 [m,H-C(5')]; 1,55 [m,H-C(4'), H-C(5')]; 1,27 [t,J=6,2, CH₃(2''')]; 1,24, 1,22, 1,18, 1,16, 1,15, 1,14 [6s, CH₃-C(1'), CH₃-C(1"), CH₃(2")].
¹³C-NMR (75,5 MHz, CDCl₃): 167,3/166,4 [C(1)]; 152,4/151,1 [C(3)]; 141,8/140,4 [C(5)]; 134,6/128,9 [C(4)]; 117,9/115,9 [C(2)]; 85,5/85,4 [C(1')]; 73,3/73,2 [C(1'')]; 59,6/59,5 [C(1''')]; 57,7/57,4 [C(2')]; 54,6/54,3 [C(3')]; 40,5 [C(5')]; 28,3/28,2 [C(2')]; 27,7/27,2 [CH₃-C(1')]; 26,1/25,9 [CH₃-C(1")]; 25,4/25,2 [C(4')]; 21,2 [CH₃-C(3)]; 14,3/13,8 [C(2''')].
IR (CHCl₃): 3550w, 2965s, 2875m, 2455w, 1690s, 1630s, 1610s, 1450m, 1375s, 1350m, 1250s, 1150s, 1090s, 1040s, 1015m, 1000m, 975m, 940m.
MS (EI, 70 eV, 150°C): 368 (M⁺,98); 353 (12); 335 (11); 309 (98), 278 (21),263 (21); 232 (15); 174 (20); 143 (100); 73 (34).

### Beispiel 11

### Reduktion und Entschützung XIV → XV

200 mg (0,54 mmol) 5-[5-(1-Hydroxy-1-methylethyl)-2-methyl-2-trimethylsilyloxy-cyclopentyl]-3-methyl-penta-2,4-diensäure-ethylester wurden in 3 ml Hexan unter Argon auf -65°C abgekühlt, und 4 ml einer 1M-Lösung von Diisobutylaluminiumhydrid wurden so zugespritzt, dass die Temperatur des Reaktionsgemisches nicht über -60°C stieg. Die Lösung wurde innert einer Stunde auf Raumtemperatur erwärmt. Dann wurden 3 ml gesättigte Ammoniumchloridlösung sorgfältig zugespritzt, die abgetrennte wässrige Phase dreimal mit Ethylacetat extrahiert und die vereinigten organischen Phasen eingedampft, in 6 ml Tetrahydrofuran gelöst und mit 0,5 ml 2M Salzsäure versetzt. Die resultierende Lösung wurde 30 Minuten gerührt, zwischen Ethylacetat und Wasser verteilt und die abgetrennte wässrige Phase dreimal mit Ethylacetat extrahiert. Man trocknete die vereinigten organischen Phasen über wasserfreiem Magnesiumsulfat, dampfte sie ein und unterwarf den Rückstand der Flash-Säulenchromatographie unter Verwendung von Silikagel und einem Gemisch von 30-100% Ethylacetat in Hexan.

Auf diese Weise erhielt man 20 mg (14,5% der theoretischen Ausbeute) 5-[2-Hydroxy-5-(1-hydroxy-methylethyl)-2-methyl-cyclopentyl]-3-methylpenta-2,4-dien-1-ol als weissen Festkörper.
¹H-NMR (300 MHz, Dimethylsulfoxid): 6,27 [d,J=15,8,H-C(4)]; 5,94 [d,J=15,8,H-C(4)]; 5,67 [dd,J=15,6; 8,4,H-C(5)]; 5,60 [dd,J=15,8; 8,6,H-C(5)]; 5,43 [t,J=6,6,H-C(2)]; 5,30 [t,J=6,6,H-C(2)]; 4,53 [t,J=6,6,OH]; 4,05 [m,H₂-C(1)]; 3,88 [t,J=6,6;OH]; 3,33 [s,OH]; 2,03 [m,H-C(2'),H-C(3')]; 1,76 [s,CH₃-C(3)]; 1,75 [m,H-C(5')]; 1,68 [s,CH₃-C(3)]; 1,56 [m,H-C(4'), H-C(5')]; 1,44 [m,H-C(4')]; 1,06/1,05/1,01/0,98/0,97 [5s,CH₃-C(1'),CH₃-C(1"),CH₃(2")].
¹³C-NMR (75,5 MHz, Dimethylsulfoxid): 135,1/132,2 [C(5)]; 134,7/127,3 [C(4)]; 129,9/128,4 [C(2)]; 57,7/56,9 [C(1)]; 55,6/55,4 [C(2')]; 53,9/53,8 [C(3')]; 40,4/40,3 [C(5')]; 29,9/29,8/27,8/27,6/26,2 [CH₃-C(1'),CH₃-C(1"),C(2")]; 24,5 [C(4')]; 20,6/12,6 [CH₃-C(3)].
IR (CHCl₃):3685m, 3610m, 3420m, 3010s, 2970s, 2925m, 1605m, 1515w, 1420m, 1380m, 1230s, 1050m, 1030m, 1010m, 975w, 930m.
MS (EI, 70 eV, 150°C): 236 (M⁺-18,18); 218 (14); 203 (12); 178 (61), 160 (25),145 (25); 120 (100); 105 (39); 93 (22); 59 (18); 43 (24).

### Beispiel 12

### Phosphoniumsalzbildung XV → X

20 mg (0,078 mmol) 5-[2-Hydroxy-5-(1-hydroxy-1-methylethyl)-2-methylcyclopentyl]-3-methyl-penta-2,4-dien-1-ol und 30 mg (0,086 mmol) Triphenylphosphoniumbromid wurden in 2 ml Methanol gelöst, und die Lösung wurde 29 Stunden bei Raumtemperatur unter Stickstoff und unter Lichtausschluss gerührt. Danach wurde das Reaktionsgemisch in etwa 100 ml eiskalten tert.Butylmethylether eingeführt und auf diese Weise das produzierte Phosphoniumsalz ausgefällt. Nach Abdekantierung des Ueberstandes und Filtration wurde der gesammelte Niederschlag mit tert.Butylmethylether gewaschen und unter vermindertem Druck getrocknet.

Auf diese Weise erhielt man 29,3 mg (65% der theoretischen Ausbeute) (5-[2-Hydroxy-5-(1-hydroxy-1-methylethyl)-2-methyl-cyclopentyl]-3-methylpenta-2,4-dienyl)triphenylphosphoniumbromid als weissen Festkörper. Dieses Produkt kann weiter gemäss den Beispielen 8 und 9 in das 2,6-Cyclolycopin-1,5-diol übergeführt werden.

## Patentansprüche

1. Verfahren zur Herstellung des Lycopinmetaboliten 2,6-Cyclolycopin-1,5-diol der Formel 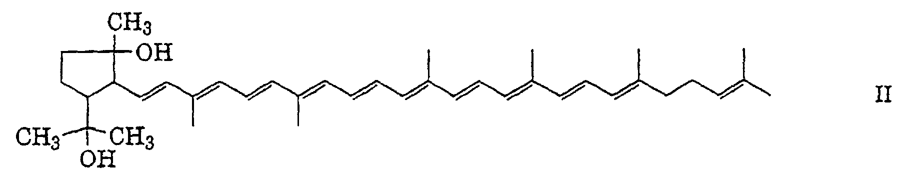 **dadurch gekennzeichnet, dass** man α-Terpinylacetat der Formel 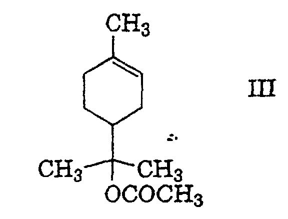 zu 4-(1-Acetoxy-1-methylethyl)-1-methyl-cyclohexan-1,2-diol der Formel 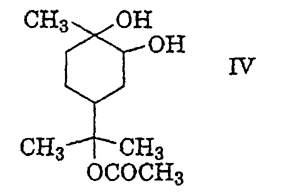 [Cyclohexandiol (IV)] oxidativ dihydroxyliert, das Cyclohexandiol (IV) zu 3-(1-Acetoxy-1-methylethyl)-6-oxo-heptanal der Formel 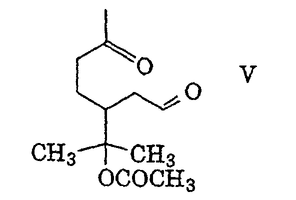 [Ketoaldehyd (V)] oxidativ spaltet, den Ketoaldehyd (V) einer intramolekularen Aldolkondensation zu 3-(1-Acetoxy-1-methylethyl)-2-formyl-1-methylcyclopentanol der Formel 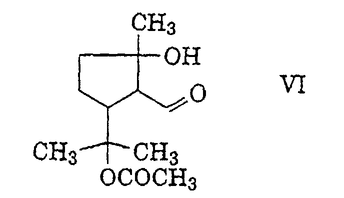 [Cyclopentanol (VI)] unterwirft, das Cyclopentanol (VI) zu 3-(1-Acetoxy-1-methylethyl)-2-formyl-1-methyl-1-trimethylsilyloxy-cyclopentan der Formel 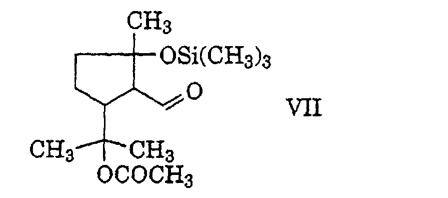 [Formylcyclopentan (VII)] silyliert, das Formylcyclopentan (VII) einer C₃-Kettenverlängerung mit Aceton sowie gleichzeitig einer Verseifung zur Abspaltung der Acetylgruppe zu 4-[5-(1-Hydroxy-1-methylethyl)-2-methyl-2-trimethylsilyloxy-cyclopentyl]-3-buten-2-on der Formel 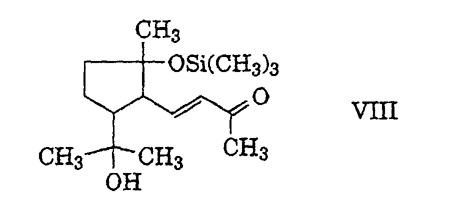 [Cyclopentylbutenon (VIII)] unterwirft, das Cyclopentylbutenon (VIII) mit Vinylmagnesiumbromid zu 5-[5-(1-Hydroxy-1-methylethyl)-2-methyl-2-trimethylsilyloxy-cyclopentyl]-3-methyl-penta-1,4-dien-3-ol der Formel 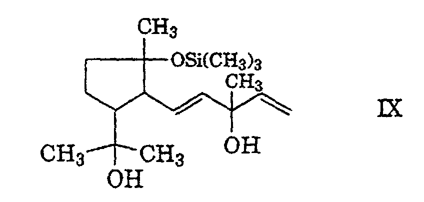 [Pentadienol (IX)] umsetzt, das Pentadienol (IX) unter Entschützung der silylierten Hydroxygruppe in das (5-[2-Hydroxy-5-(1-hydroxy-1-methylethyl)-2-methyl-cyclopentyl]-3-methyl-penta-2,4-dienyl)triphenylphosphoniumsalz der Formel 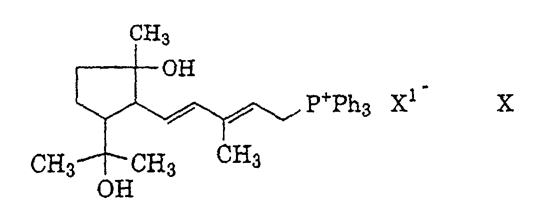 , worin Ph Phenyl und X¹⁻ Halogenid oder Hydrogensulfat bedeuten,
[Phosphoniumsalz (X)] überführt, das Phosphoniumsalz (X) mit 2,7-Dimethyl-2,4,6-octatrien-1,8-dial der Formel 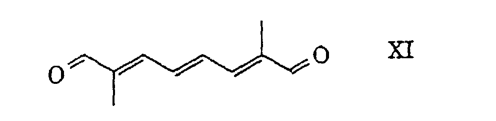 [C₁₀-Dial (XI)] einer Wittig-Reaktion zu 2,7,11-Trimethyl-13-[2-hydroxy-5-(1-hydroxy-1-methylethyl)-2-methyl-cyclopentyl]-trideca-2,4,6,8,10,12-hexaenal der Formel 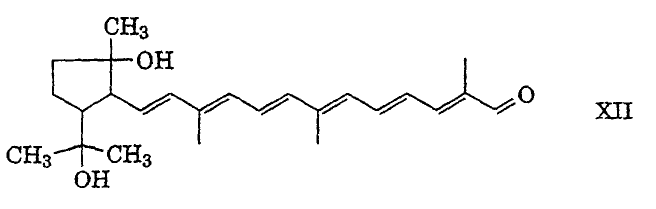 [Tridecahexaenal (XII)] unterwirft und das Tridecahexaenal (XII) mit einem (3,7,11-Trimethyl-dodeca-2,4,6,10-tetraenyl)triphenylphosphoniumsalz der Formel 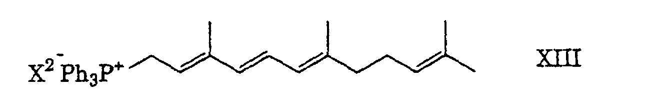 , worin Ph Phenyl und X²⁻ Halogenid oder Hydrogensulfat bedeuten,
[Phosphoniumsalz (XIII)] einer Wittig-Reaktion zum erwünschten 2,6-Cyclolycopin-1,5-diol der Formel II unterwirft.

2. Verfahren zur Herstellung des Lycopinmetaboliten 2,6-Cyclolycopin-1,5-diol der Formel 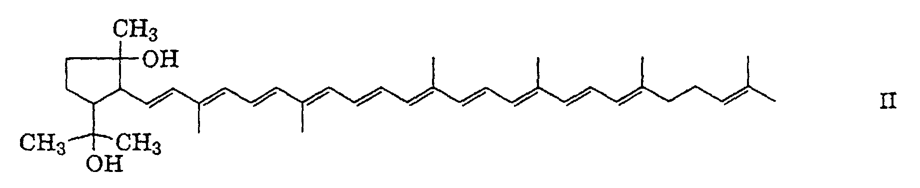 **dadurch gekennzeichnet, dass** man α-Terpinylacetat der Formel 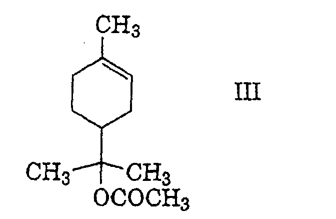 zu 4-(1-Acetoxy-1-methylethyl)-1-methyl-cyclohexan-1,2-diol der Formel 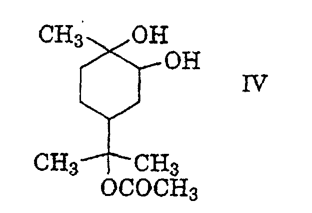 [Cyclohexandiol (IV)] oxidativ dihydroxyliert, das Cyclohexandiol (IV) zu 3-(1-Acetoxy-1-methylethyl)-6-oxo-heptanal der Formel 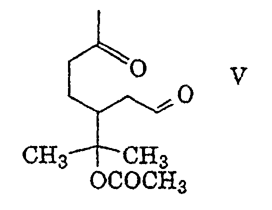 [Ketoaldehyd (V)] oxidativ spaltet, den Ketoaldehyd (V) einer intramolekularen Aldolkondensation zu 3-(1-Acetoxy-1-methylethyl)-2-formyl-1-methylcyclopentanol der Formel 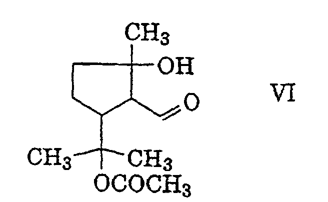 [Cyclopentanol (VI)] unterwirft, das Cyclopentanol (VI) zu 3-(1-Acetoxy-1-methylethyl)-2-formyl-1-methyl-1-trimethylsilyloxy-cyclopentan der Formel 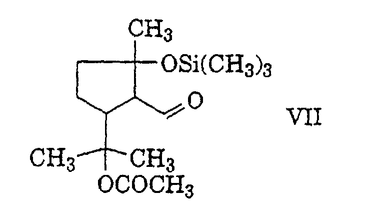 [Formylcyclopentan (VII)] silyliert, das Formylcyclopentan (VII) einer C₃-Kettenverlängerung mit Aceton sowie gleichzeitig einer Verseifung zur Abspaltung der Acetylgruppe zu 4-[5-(1-Hydroxy-1-methylethyl)-2-methyl-2-trimethylsilyloxy-cyclopentyl]-3-buten-2-on der Formel 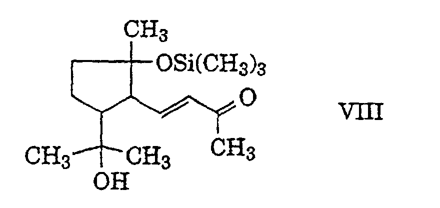 [Cyclopentylbutenon (VIII)] unterwirft, das Cyclopentylbutenon (VIII) mit einem Phosphonoessigsäure-trialkylester in Gegenwart einer Base einer Horner-Emmons-Olefinierung zum entsprechenden 5-[5-(1-Hydroxy-1-methylethyl)-2-methyl-2-trimethylsilyloxy-cyclopentyl]-3-methyl-penta-2,4-diensäure-alkylester der Formel 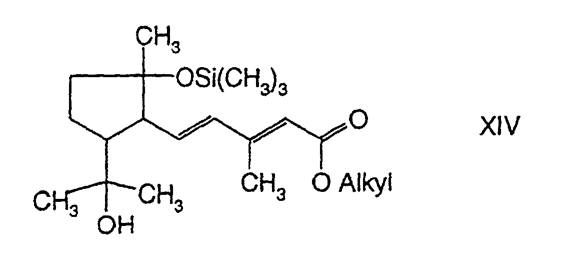 , worin Alkyl C₁₋₆-Alkyl bedeutet,
[Pentadiensäureester (XIV)] unterwirft, den Pentadiensäureester (XIV) unter Entschützung der silylierten Hydroxygruppe zum 5-[2-Hydroxy-5-(1-hydroxy-1-methylethyl)-2-methyl-cyclopentyl]-3-methyl-penta-2,4-dien-1-ol der Formel 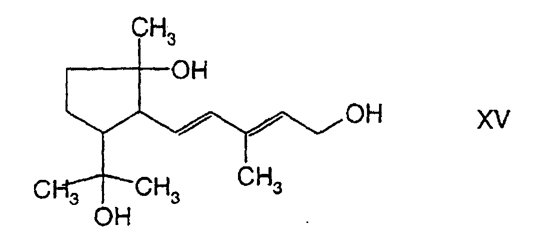 [Pentadienol (XV)] reduziert, das Pentadienol (XV) in das (5-[2-Hydroxy-5-(1-hydroxy-1-methylethyl)-2-methyl-cyclopentyl]-3-methyl-penta-2,4-dienyl)trzphenylphosphoniumsalz der Formel 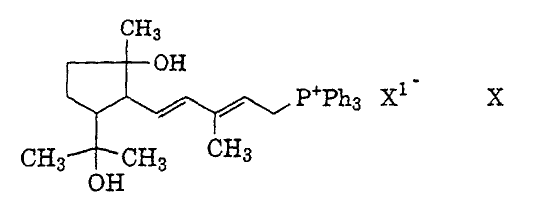 , worin Ph Phenyl und X¹⁻ Halogenid oder Hydrogensulfat bedeuten,
[Phosphoniumsalz (X)] überführt, das Phosphoniumsalz (X) mit 2,7-Dimethyl-2,4,6-octatrien-1,8-dial der Formel 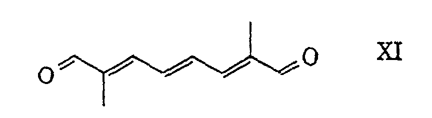 [C₁₀-Dial (XI)] einer Wittig-Reaktion zu 2,7,11-Trimethyl-13-[2-hydroxy-5-(1-hydroxy-1-methylethyl)-2-methyl-cyclopentyl]-trideca-2,4,6,8, 10,12-hexaenal der Formel 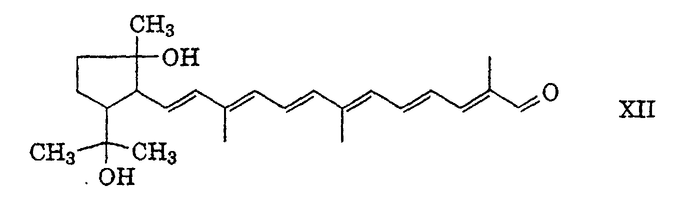 [Tridecahexaenal (XII)] unterwirft und das Tridecahexaenal (XII) mit einem (3,7,11-Trimethyl-dodeca-2,4,6,10-tetraenyl)triphenylphosphoniumsalz der Formel 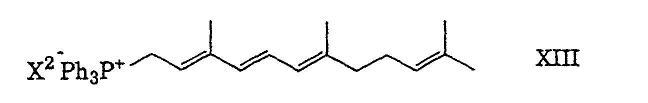 , worin Ph Phenyl und X²⁻ Halogenid oder Hydrogensulfat bedeuten,
[Phosphoniumsalz (XIII)] einer Wittig-Reaktion zum erwünschten 2,6-Cyclolycopin-1,5-diol der Formel II unterwirft.

3. 3-(1-Acetoxy-1-methylethyl)-6-oxo-heptanal.

4. 3-(1-Acetoxy-1-methylethyl)-2-formyl-1-methyl-cyclopentanol.

5. 3-(1-Acetoxy-1-methylethyl)-2-formyl-1-methyl-1-trimethylsilyloxycyclopentan.

6. 4-[5-(1-Hydroxy-1-methylethyl)-2-methyl-2-trimethylsilyloxycyclopentyl]-3-buten-2-on.

7. 5-[5-(1-Hydroxy-1-methylethyl)-2-methyl-2-trimethylsilyloxycyclopentyl]-3-methyl-penta-1,4-dien-3-ol.

8. Der 5-[5-(1-Hydroxy-1-methylethyl)-2-methyl-2-trimethylsilyloxycyclopentyl3-3-methyl-penta-2,4-diensäure-alkylester der Formel 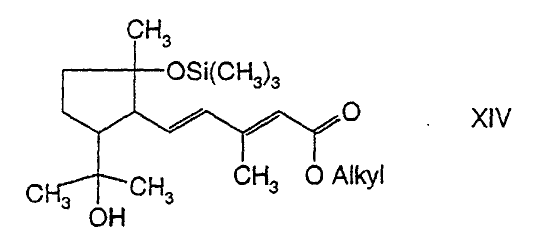 , worin Alkyl C₁₋₆-Alkyl bedeutet.

9. 5-[2-Hydroxy-5-(1-hydroxy-1-methylethyl)-2-methyl-cyclopentyl]-3-methyl-penta-2,4-dien- 1-ol.

10. Das (5-[2-Hydroxy-5-(1-hydroxy-1-methylethyl)-2-methyl-cyclopentyl]-3-methyl-penta-2,4-dienyl)triphenylphosphoniumsalz der Formel 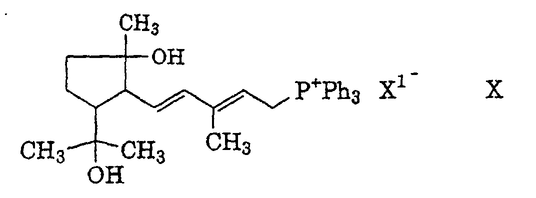 , worin Ph Phenyl und X¹⁻ Halogenid oder Hydrogensulfat bedeuten.

11. 2,7,11-Trimethyl-13-[2-hydroxy-5-(1-hydroxy-1-methylethyl)-2-methyl-cyclopentyl]-trideca-2,4,6,8,10,12-hexaenal.

## Claims

1. A process for the manufacture of the lycopene metabolite 2,6-cyclolycopene-1,5-diol of the formula 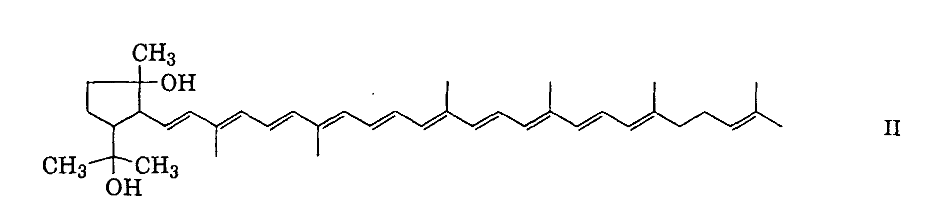 which process comprises oxidatively dihydroxylating α-terpinyl acetate of the formula 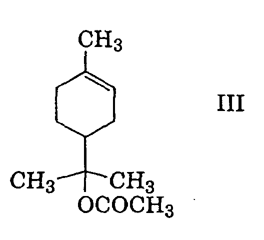 to 4-(1-acetoxy-1-methylethyl)-1-methyl-cyclohexane-1,2-diol of the formula 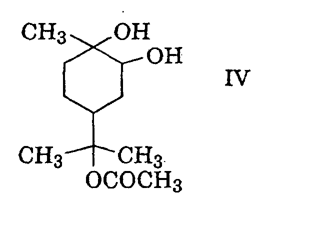 [cyclohexanediol (IV)], oxidatively cleaving the cyclohexanediol (IV) to 3-(1-acetoxy-1-methylethyl)-6-oxo-heptanal of the formula 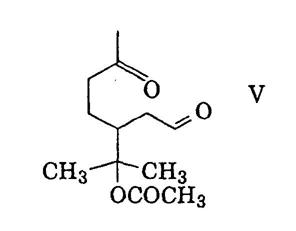 [ketoaldehyde (V)], subjecting the ketoaldehyde (V) to an intramolecular aldol condensation to 3-(1-acetoxy-1-methylethyl)-2-formyl-1-methyl-cyclopentanol of the formula 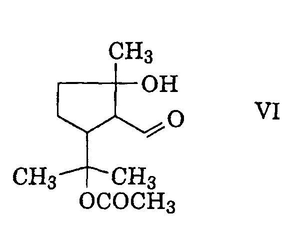 [cyclopentanol (VI)], silylating the cyclopentanol (VI) to 3-(1-acetoxy-1-methylethyl)-2-formyl-1-methyl-1-trimethylsilyloxy-cyclopentane of the formula 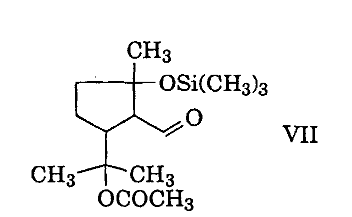 [formylcyclopentane (VII)], subjecting the formylcyclopentane (VII) to a C₃-chain lengthening with acetone and simultaneously to a saponification for the cleavage of the acetyl group to give 4-[5-(1-hydroxy-1-methylethyl)-2-methyl-2-trimethylsilyloxycyclopentyl]-3-buten-2-one of the formula 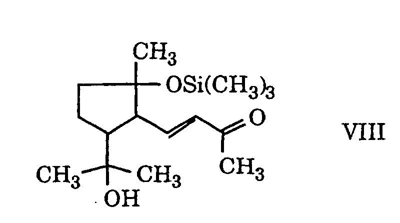 [cyclopentylbutenone (VIII)], reacting the cyclopentylbutanene (VIII) with vinylmagnesium bromide to give 5-[5-(1-hydroxy-1-methylethyl)-2-methyl-2-trimethylsilyloxycyclopentyl]-3-methyl-penta-1,4-dien-3-ol of the formula 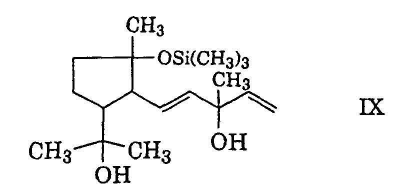 [pentadienol (IX)], converting the pentadienol (IX) with deprotection of the silylated hydroxy group into the (5-[2-hydroxy-5-(1-hydroxy-1-methylethyl)-2-methyl-cyclopentyl]-3-methyl-penta-2,4-dienyl)triphenylphosphonium salt of the formula 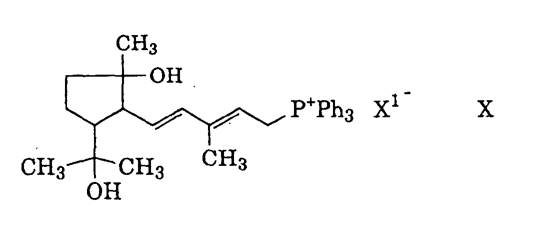 wherein Ph signifies phenyl and X¹⁻ signifies halide or hydrogen sulphate,
[phosphonium salt (X)], subjecting the phosphonium salt (X) to a Wittig reaction with 2,7-dimethyl-2,4,6-octatriene-1,8-dial of the formula 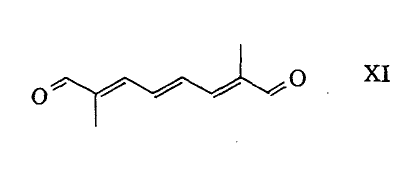 [C₁₀-dial (XI)] to give 2,7,11-trimethyl-13-[2-hydroxy-5-(1-hydroxy-1-methylethyl)-2-methyl-cyclopentyl]-trideca-2,4,6,8,10,12-hexaenal of the formula 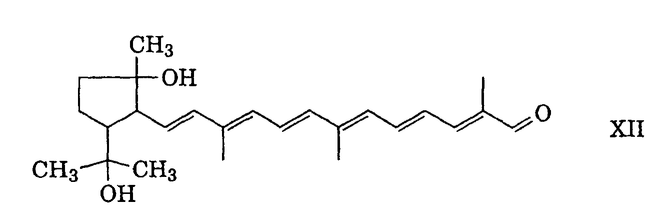 [tridecahexaenal (XII)] and subjecting the tridecahexaenal (XII) to a Wittig reaction with a (3,7,11-trimethyl-dodeca-2,4,6,10-tetraenyl)triphenylphosphonium salt of the formula 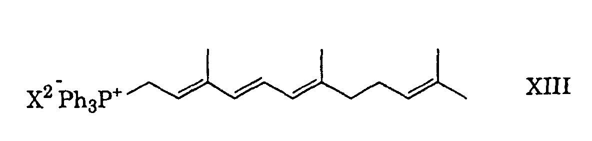 wherein Ph signifies phenyl and X²⁻ signifies halide or hydrogen sulphate,
[phosphonium salt (XIII)] to give the desired 2,6-cyclolycopene-1,5-diol of formula II.

2. A process for the manufacture of the lycopene metabolite 2,6-cyclolycopene-1,5-diol of the formula 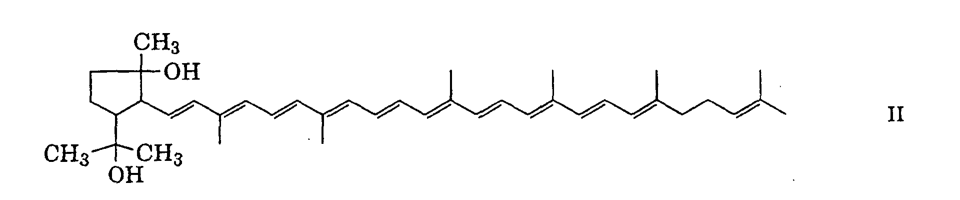 which process comprises oxidatively dihydroxylating α-terpinyl acetate of the formula 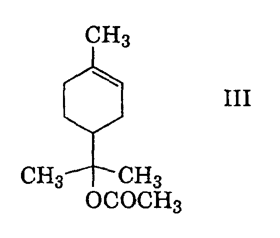 to 4-(1-acetoxy-1-methylethyl)-1-methyl-cyclohexane-1,2-diol of the formula 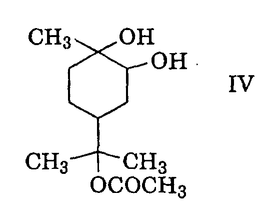 [cyclohexanediol (IV)], oxidatively cleaving the cyclohexanediol (IV) to 3-(1-acetoxy-1-methylethyl)-6-oxo-heptanal of the formula 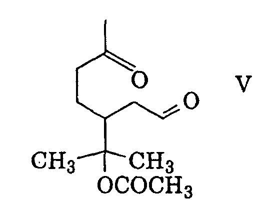 [ketoaldehyde (V)], subjecting the ketoaldehyde (V) to an intramolecular aldol condensation to 3-(1-acetoxy-1-methylethyl)-2-formyl-1-methyl-cyclopentanol of the formula 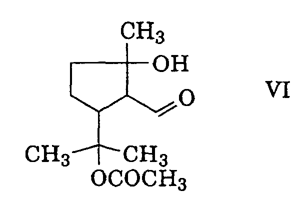 [cyclopentanol (VI)], silylating the cyclopentanol (VI) to 3-(1-acetoxy-1-methylethyl)-2-formyl-1-methyl-1-trimethylsilyloxy-cyclopentane of the formula 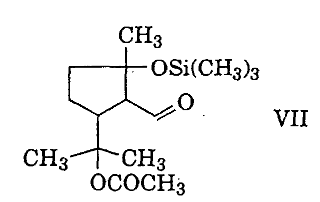 [formylcyclopentane (VII)], subjecting the formylcyclopentane (VII) to a C₃-chain lengthening with acetone and simultaneously to a saponification for the cleavage of the acetyl group to give 4-[5-(1-hydroxy-1-methylethyl)-2-methyl-2-trimethylsilyloxycyclopentyl]-3-buten-2-one of the formula 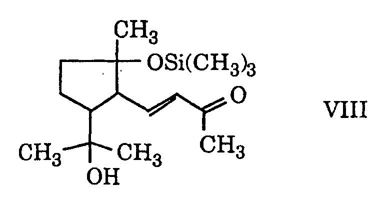 [cyclopentylbutenone (VIII)], subjecting the cyclopentylbutenone (VIII) to a Horner-Emmons olefination with a trialkyl phosphonoacetate in the presence of a base to give the corresponding alkyl 5-[5-(1-hydroxy-1-methylethyl)-2-methyl-2-trimethylsilyloxycyclopentyl]-3-methyl-penta-2,4-dienoate of the formula 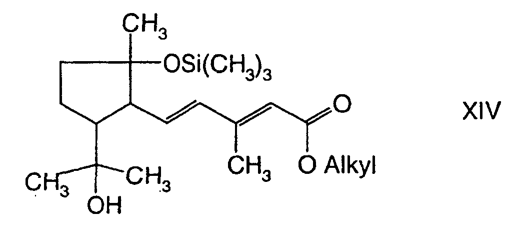 wherein Alkyl signifies C₁₋₆-alkyl,
[pentadienoic acid ester (XIV)], reducing the pentadienoic acid ester (XIV) with deprotection of the silylated hydroxy group to give 5-[2-hydroxy-5-(1-hydroxy-1-methylethyl)-2-methyl-cyclopentyl]-3-methyl-penta-2,4-dien-1-ol of the formula 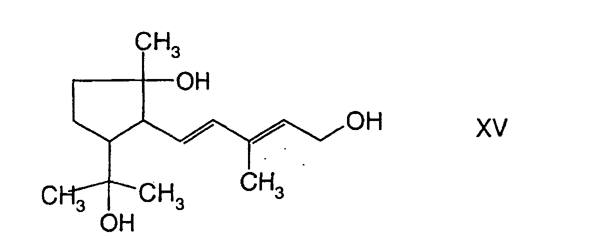 [pentadienol (XV)], converting the pentadienol (XV) into the (5-[2-hydroxy-5-(1-hydroxy-1-methylethyl)-2-methyl-cyclopentyl]-3-methyl-penta-2,4-dienyl)- triphenylphosphonium salt of the formula 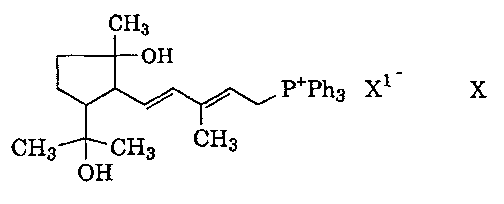 wherein Ph signifies phenyl and X¹⁻ signifies halide or hydrogen sulphate,
[phosphonium salt (X)], subjecting the phosphonium salt (X) to a Wittig reaction with 2,7-dimethyl-2,4,6-octatriene-1,8-dial of the formula 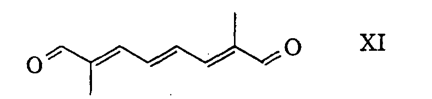 [C₁₀-dial (XI)] to give 2,7,11-trimethyl-13-[2-hydroxy-5-(1-hydroxy-1-methylethyl)-2-methyl-cyclopentyl]trideca-2,4,6,8,10,12-hexaenal of the formula 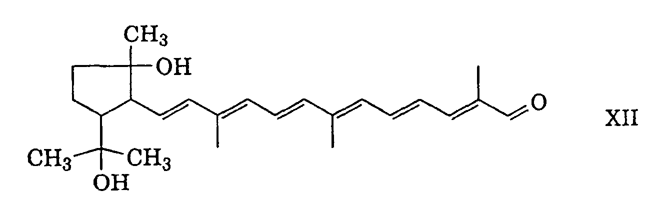 [tridecahexaenal (XII)] and subjecting the tridecahexaenal (XII) to a Wittig reaction with a (3,7,11-trimethyl-dodeca-2,4,6,10-tetraenyl)triphenylphosphonium salt of the formula 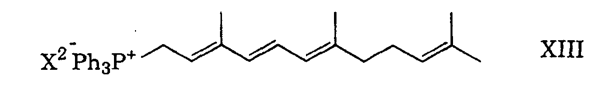 wherein Ph signifies phenyl and X²⁻ signifies halide or hydrogen sulphate,
[phosphonium salt (XIII)] to give the desired 2,6-cyclolycopene-1,5-diol of formula II.

3. 3-(1-Acetoxy-1-methylethyl)-6-oxo-heptanal.

4. 3-(1-Acetoxy-1-methylethyl)-2-formyl-1-methyl-cyclopentanol.

5. 3-(1-Acetoxy-1-methylethyl)-2-formyl-1-methyl-1-trimethylsilyloxycyclopentane.

6. 4-[5-(1-Hydroxy-1-methylethyl)-2-methyl-2-trimethylsilyloxy-cyclopentyl]-3-buten-2-one.

7. 5-[5-(1-Hydroxy-1-methylethyl)-2-methyl-2-trimethylsilyloxy-cyclopentyl]-3-methyl-penta-1,4-dien-3-ol.

8. An alkyl 5-[5-(1-hydroxy-1-methylethyl)-2-methyl-2-trimethylsilyloxycyclopentyl]-3-methyl-penta-2,4-dienoate of the formula 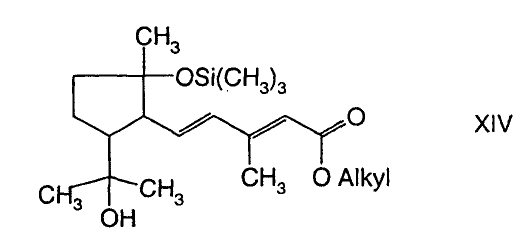 wherein Alkyl signifies C₁₋₆-alkyl.

9. 5-[2-Hydroxy-5-(1-hydroxy-1-methylethyl)-2-methyl-cyclopentyl]-3-methyl-penta-2,4-dien-1-ol.

10. A (5-[2-hydroxy-5-(1-hydroxy-1-methylethyl)-2-methyl-cyclopentyl]-3-methyl-penta-2,4-dienyl)triphenylphosphonium salt of the formula 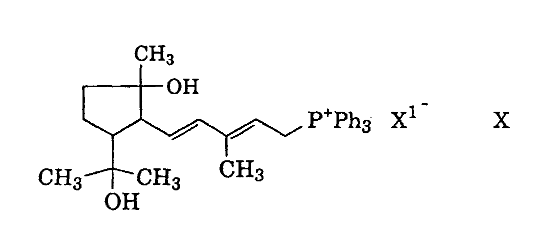 wherein Ph signifies phenyl and X¹⁻ signifies halide or hydrogen sulphate.

11. 2,7,11-Trimethyl-13-[2-hydroxy-5-(1-hydroxy-1-methylethyl)-2 methylcyclopentyl]-trideca-2,4,6,8,10,12-hexaenal.

## Revendications

1. Procédé pour la préparation du métabolite de lycopène 2,6-cyclolycopène-1,5-diol de formule 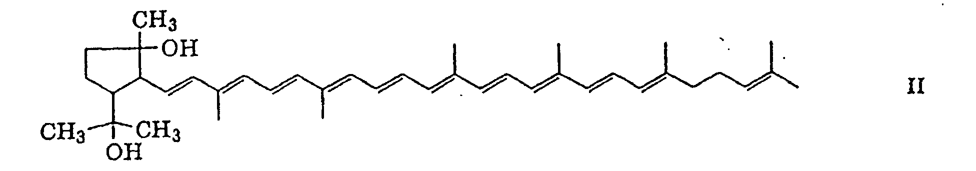 **caractérisé en ce qu'**on soumet à une dihydroxylation par oxydation l'α-acétate de terpinyle de formule 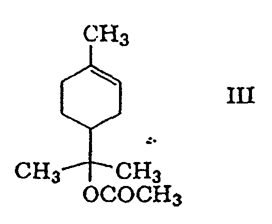 pour aboutir au 4-(1-acétoxy-1-méthyléthyl)-1-méthylcyclohexane-1,2-diol de formule 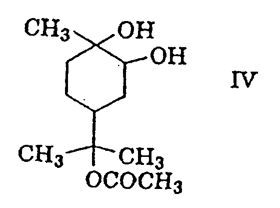 [cyclohexanediol (IV)], on ouvre par oxydation le cyclohexanediol (IV) pour obtenir le 3-(1-acétoxy-1-méthyléthyl)-6-oxoheptanal de formule 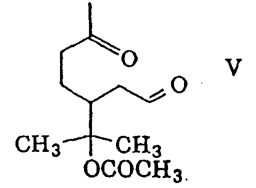 [cétoaldéhyde (V)], on soumet le cétoaldéhyde (V) à une condensation d'aldol intramoléculaire pour aboutir au 3-(1-acétoxy-1-méthyléthyl)-2-formyl-1-méthylcyclopentanol de formule 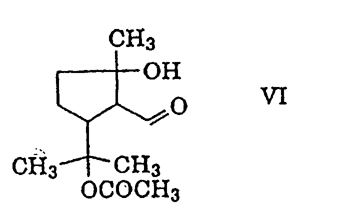 [cyclopentanol (VI)], le cyclopentanol (VI) est silylé en 3-(1-acétoxy-1-méthyléthyl)-2-formyl-1-méthyl-1-triméthylsilyloxycyclopentane de formule 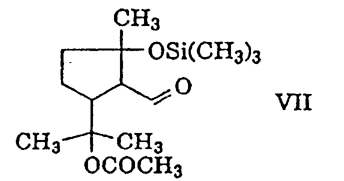 [formylcyclopentane (VII)], on soumet le formylcyclopentane (VII) à une extension de chaîne en C₃ avec de l'acétone ainsi qu'en même temps à une saponification pour l'élimination du groupe acétyle, conduisant à la 4-[5-(1-hydroxy-1-méthyléthyl)-2-méthyl-2-triméthylsilyloxycyclopentyl]-3-butén-2-one de formule 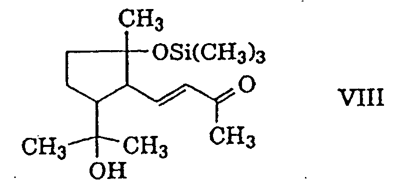 [cyclopentylbuténone (VIII)], on fait réagir la cyclopentylbuténone (VIII) avec du bromure de vinylmagnésium, pour aboutir au 5-[5-(1-hydroxy-1-méthyléthyl)-2-méthyl-2-triméthylsilyloxycyclopentyl]-3-méthylpenta-1,4-dién-3-ol de formule 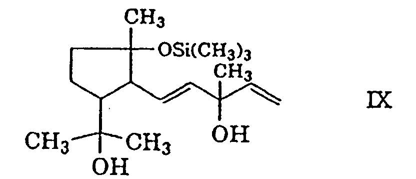 [pentadiénol (IX)], on convertit le pentadiénol (IX), avec déprotection du groupe hydroxy silylé, en le sel de (5-[2-hydroxy-5-(1-hydroxy-1-méthyléthyl)-2-méthylcyclopentyl]-3-méthylpenta-2,4-diényl)triphénylphosphonium de formule 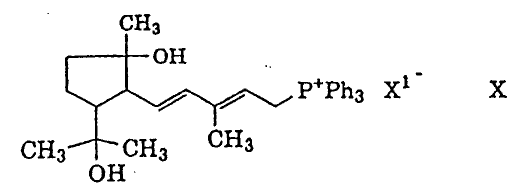 dans laquelle Ph signifie le groupe phényle et X¹⁻ représente un ion halogénure ou hydrogénosulfate [sel de phosphonium (X)], on soumet le sel de phosphonium (X) à une réaction de Wittig avec du 2,7-diméthyl-2,4,6-octatriène-1,8-dial de formule 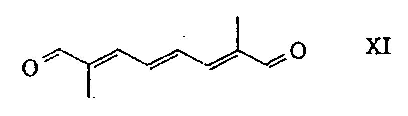 [dial en C₁₀ (XI)], pour aboutir au 2,7,11-triméthyl-13-[2-hydroxy-5-(1-hydroxy-1-méthyléthyl)-2-méthylcyclopentyl]tridéca-2,4,6,8,10,12-hexaénal de formule 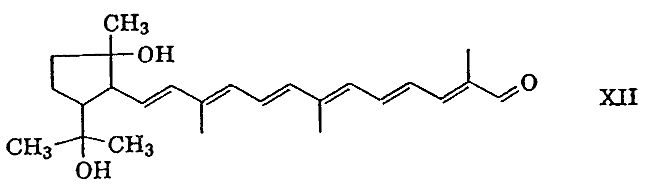 [tridécahexaénal (XII)], et on soumet le tridécahexaénal (XII) à une réaction de Wittig avec un sel de (3,7,11-triméthyldodéca-2,4,6,10-tétraényl)triphénylphosphonium de formule 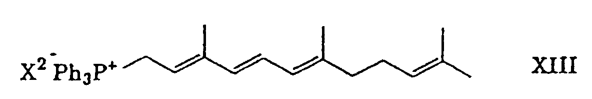 dans laquelle Ph signifie le groupe phényle et X²⁻ représente un ion halogénure ou hydrogénosulfate [sel de phosphonium (XIII)], pour aboutir au 2,6-cyclolycopène-1,5-diol recherché de formule II.

2. Procédé pour la préparation du métabolite de lycopène-2,6-cyclolycopène-1,5-diol de formule 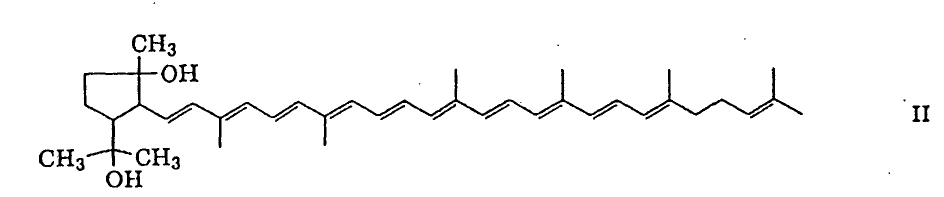 **caractérisé en ce qu'**on soumet à une dihydroxylation par oxydation l'α-acétate de terpinyle de formule 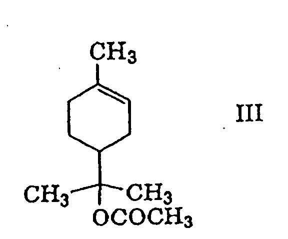 pour aboutir au 4-(1-acétoxy-1-méthyléthyl)-1-méthylcyclohexane-1,2-diol de formule 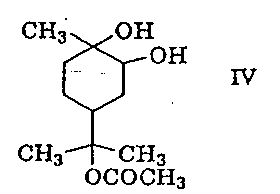 [cyclohexanediol (IV)], on ouvre par oxydation le cyclohexanediol (IV) pour aboutir au 3-(1-acétoxy-1-méthyléthyl)-6-oxoheptanal de formule 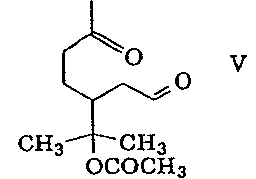 [cétoaldéhyde (V)], on soumet le cétoaldéhyde (V) à une condensation d'aldol intramoléculaire pour aboutir au 3-(1-acétoxy-1-méthyléthyl)-2-formyl-1-méthylcyclopentanol de formule 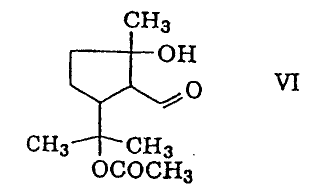 [cyclopentanol (VI)], le cyclopentanol (VI) est silylé en 3-(1-acétoxy-1-méthyléthyl)-2-formyl-1-méthyl-1-triméthylsilyloxycyclopentane de formule 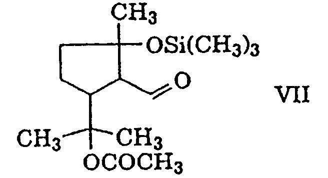 [formylcyclopentane (VII)], on soumet le formylcyclopentane (VII) à une extension de chaîne en C₃ avec de l'acétone ainsi qu'en même temps à une saponification pour l'élimination du groupe acétyle, conduisant à la 4-[5-(1-hydroxy-1-méthyléthyl)-2-méthyl-2-triméthylsilyloxycyclopentyl]-3-butén-2-one de formule 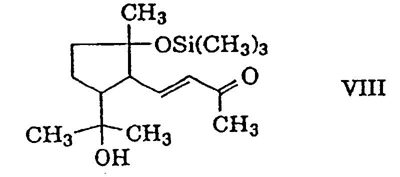 [cyclopentylbuténone (VIII)], on soumet la cyclopentylbuténone (VIII) à une oléfination de Horner-Emmons avec un phosphonoacétate de trialkyle en présence d'une base, pour aboutir au 5-[5-(1-hydroxy-1-méthyléthyl)-2-méthyl-2-triméthylsilyloxycyclopentyl]-3-méthylpenta-2,4-diénoate d'alkyle correspondant de formule 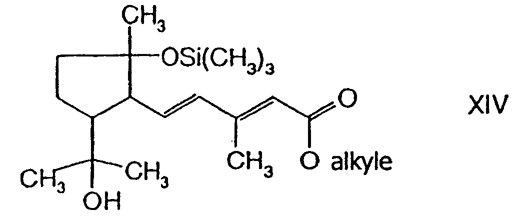 dans laquelle alkyle signifie un groupe alkyle en C₁₋₆,
[ester d'acide pentadiénoïque (XIV)], on réduit l'ester d'acide pentadiénoïque (XIV), avec déprotection du groupe hydroxy silylé, en le 5-[2-hydroxy-5-(1-hydroxy-1-méthyléthyl)-2-méthylcyclopentyl]-3-méthylpenta-2,4-dién-1-ol de formule 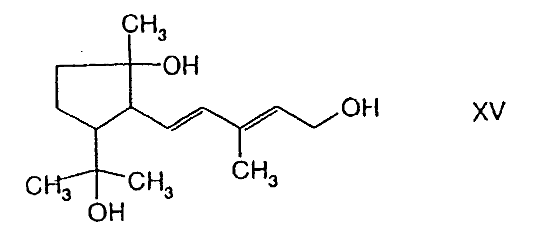 [pentadiénol (XV)], on convertit le pentadiénol (XV) en le sel de (5-[2-hydroxy-5-(1-hydroxy-1-méthyléthyl)-2-méthylcyclopentyl]-3-méthylpenta-2,4-diényl)triphénylphosphonium de formule 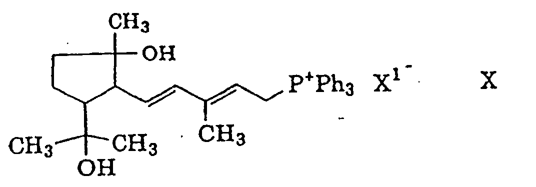 dans laquelle Ph signifie le groupe phényle et X¹⁻ représente un ion halogénure ou hydrogénosulfate [sel de phosphonium (X)], on soumet le sel de phosphonium (X) à une réaction de Wittig avec le 2,7-diméthyl-2,4,6-octatriène-1,8-dial de formule 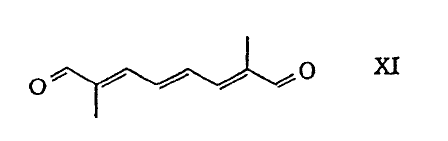 [dial en C₁₀ (XI)], conduisant au 2,7,11-triméthyl-13-[2-hydroxy-5-(1-hydroxy-1-méthyléthyl)-2-méthylcyclopentyl]tridéca-2,4,6,8,10,12-hexaénal de formule 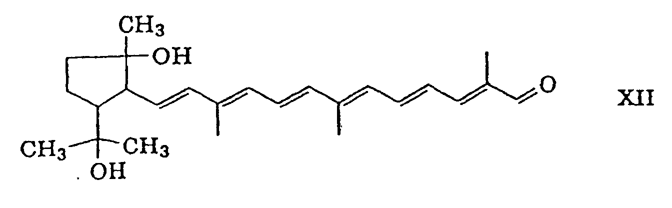 [tridécahexaénal (XII)], et on soumet le tridécahexaénal (XII) à une réaction de Wittig avec un sel de (3,7,11-triméthyldodéca-2,4,6,10-tétraényl)triphénylphosphonium de formule 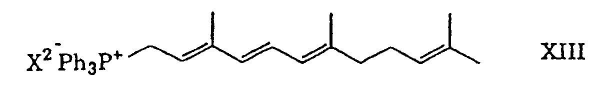 dans laquelle Ph signifie le groupe phényle et X²⁻ représente un ion halogénure ou hydrogénosulfate [sel de phosphonium (XIII)], conduisant au 2,6-cyclolycopène-1,5-diol recherché de formule II.

3. 3-(acétoxy-1-méthyléthyl)-6-oxoheptanal.

4. 3-(1-acétoxy-1-méthyléthyl)-2-formyl-1-méthylcyclopentanol.

5. 3-(1-acétoxy-1-méthyléthyl)-2-formyl-1-méthyl-1-triméthylsilyloxycyclopentane.

6. 4-[5-(1-hydroxy-1-méthyléthyl)-2-méthyl-2-triméthylsilyloxycyclopentyl]-3-butén-2-one.

7. 5-[5-(1-hydroxy-1-méthyléthyl)-2-méthyl-2-triméthylsilyloxycyclopentyl]-3-méthylpenta-1,4-dién-3-ol.

8. 5-[5-(1-hydroxy-1-méthyléthyl)-2-méthyl-2-triméthylsilyloxycyclopentyl]-3-méthylpenta-2,4-diénoate d'alkyle de formule 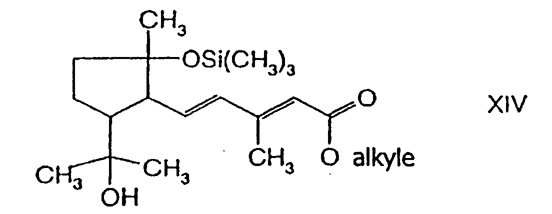 dans laquelle alkyle signifie un groupe alkyle en C₁₋₆.

9. 5-[2-hydroxy-5-(1-hydroxy-1-méthyléthyl)-2-méthylcyclopentyl]-3-méthylpenta-2,4-dién-1-ol.

10. Sel de (5-[2-hydroxy-5-(1-hydroxy-1-méthyléthyl)-2-méthylcyclopentyl]-3-méthylpenta-2,4-diényl)triphénylphosphonium de formule 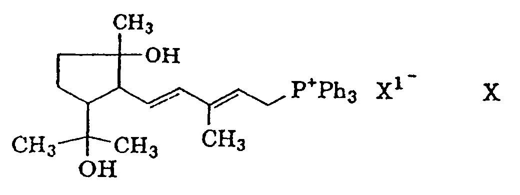 dans laquelle Ph signifie le groupe phényle et X¹⁻ représente un ion halogénure ou hydrogénosulfate.

11. 2,7,11-triméthyl-13-[2-hydroxy-5-(1-hydroxy-1-méthyléthyl)-2-méthylcyclopentyl]tridéca-2,4,6,8,10,12-hexaénal.
